# EUROPEAN PATENT SPECIFICATION

(11) **EP 3 495 372 B1**
(45) Date of publication and mention of the grant of the patent: **07.04.2021**
(21) Application number: 18207733.9
(22) Date of filing: 22.11.2018
(51) Int. Cl.: C07F 5/02, C07F 5/04, A61K 33/22, A61P 33/00, A61P 25/28, A61P 35/00

(54) **BORONATED DERIVATIVES FOR THE TREATMENT OF DISEASES AND CONDITIONS ASSOCIATED TO OXIDATIVE STRESS**
BORIERTE DERIVATE FÜR DIE BEHANDLUNG VON MIT OXIDATIVEM STRESS VERBUNDENEN KRANKHEITEN UND ZUSTÄNDEN
DÉRIVÉS BORÉS POUR LE TRAITEMENT DE MALADIES ET CONDITIONS LIÉES AU STRESS OXIDATIF

(30) Priority: 05.12.2017 IT 201700140164
(43) Date of publication of application: 12.06.2019
(73) Proprietor: Università degli Studi "G. d'Annunzio" Chieti-Pescara, 66100 Chieti (IT)
(72) Inventor: DI STEFANO, Antonio, 66100 Chieti Scalo (IT); CACCIATORE, Ivana, 66100 Chieti Scalo (CH) (IT); TURKEZ, Hasan, 25050 Erzurum (TR)
(74) Representative: Currado, Luisa

(56) References cited:
- US-A1- 2001 039 292
- MARIA KOUFAKI: "Therapeutic applications of lipoic acid: a patent review (2011 - 2014)", EXPERT OPINION ON THERAPEUTIC PATENTS., vol. 24, no. 9, 7 August 2014 (2014-08-07) , pages 993-1005, XP055456533, GB ISSN: 1354-3776, DOI: 10.1517/13543776.2014.937425
- MARVIN A SORIANO-URSUA ET AL.: "Boron-containing compounds: chemico-biological properties and expanding medicinal potential in prevention, diagnosis and therapy", EXPERT OPINION ON THERAPEUTIC PATENTS, vol. 24, no. 5, 2014, pages 485-500, XP002780451,
- SMOUM ET AL.: "Boron containing compounds as protease inhibitors", CHEMICAL REVIEWS, vol. 112, 20 April 2012 (2012-04-20), pages 4156-4220, XP002780452,

## Description

### State of the art

The present invention relates to the pharmaceutical chemistry field since it related to the design and synthesis of boron compounds having neuroprotective activity, antiplatelet activity, antitumor activity, and antimicrobial activity for the treatment of diseases and pathological conditions associated with oxidative stress.

Oxidative stress occurs when there is an imbalance between the cellular defence systems and the production of reactive oxygen (ROS) and nitrogen (RNS) species. It is known that some of the human disorders are linked to oxidative stress, such as neurological diseases (comprising Alzheimer's Disease, Parkinson's Disease, Amyotrophic Lateral Sclerosis), cancer, diseases of the circulatory system (such as atherosclerosis, stroke, ischemia), kidney diseases, lung diseases, eye diseases, and pancreatic diseases. The use of antioxidant molecules, with ROS (reactive oxygen species) and RNS (reactive nitrogen species) scavenger, direct and indirect activities activity, can avoid, retard or modify the course of these diseases. Many antioxidant molecules are under investigation to study their efficacy in the treatment of oxidative stress linked-pathologies. For example, Lipoic acid (LA) 1,2-dithiolane-3-pentanoic acid, is a natural product, soluble both in water and lipids, able to easily cross cellular membrane and blood brain barrier. Lipoic acid acts as a cofactor for several mitochondrial enzymes. Both oxidized (LA) and reduced (DHLA) forms contributes to the antioxidant properties of the molecule (Rochette L, Ghibu S, Muresan A, Vergely C. Alpha-lipoic acid: Molecular mechanisms and therapeutic potential in diabetes. Can. J. Physiol. Pharm. 2015; 93:1021-1027). Lipoic acid is a powerful antioxidant since it is a direct scavenger of reactive oxygen species (ROS) and reactive nitrogen species (RNS) that are produced following oxidative stress or age-associated disorders (Rizzo AM, Berselli P, Zava S, Montorfano G, Negroni M, Corsetto P, Berra B. Endogenous antioxidants and radical scavengers. Adv. Exp. Med. Biol. 2010; 698:52-67). Lipoic acid can increases glutathione levels through activation of nuclear factor (erythroid-derived 2)-like 2 (Nrf2). Lipoic acid regulates the homeostasis of metals by chelating copper (II), iron (II), and zinc (II) by generating stable complexes (Cacciatore I, Fornasari E, Baldassarre L, Cornacchia C, Fulle S, Di Filippo ES, Pietrangelo T, Pinnen F. A potent (R)-alpha-bis-lipoyl derivative containing 8-hydroxyquinoline scaffold: Synthesis and biological evaluation of its neuroprotective capabilities in SH-SY5Y human neuroblastoma cells. Pharmaceuticals 2013; 6:54-69). Moreover, lipoic acid shows anti-inflammatory activity by regulating the expression of proinflammatory genes and by indirectly modulating lipid levels in blood thus reducing atherosclerotic processes (Koufaki M. Therapeutic applications of lipoic acid: A patent review (2011-2014). Exp. Opin. Ther. Pat. 2014; 24:993-1005). Lipoic has a therapeutic potential in the treatment of several pathologies characterized by oxidative stress caused by elevated levels of reactive oxygen species (ROS) such as neurodegenerative diseases, such as Alzheimer's Disease, Parkinson's Disease, Amyothrophic Lateral Sclerosis and Multiple Sclerosis, cardiovascular diseases such as hypertension, atherosclerosis, ischemia reperfusion, heart failure, and diabetic vasculopathy and diabetes (Holmquist L, Stuchbury G, Berbaum K, Muscat S, Young S, Hager K, Engel J, Munch G. Lipoic acid as a novel treatment for Alzheimer's disease and related dementias. Pharm. Ther. 2007;113:154-164; De Araújo DP, Lobato RDFG, Cavalcanti JRLDP, Sampaio LRL, Araújo PVP, Silva MCC, Neves KRT, Fonteles MMDF, Sousa FCFD, Vasconcelos SMM. The contributions of antioxidant activity of lipoic acid in reducing neurogenerative progression of Parkinson's disease: A review. Int. J. Neurosci. 2011;121:51-57; Andreassen OA, Dedeoglu A, Friedlich A, Ferrante KL, Hughes D, Szabo C, Beal M. Flint Effects of an inhibitor of poly(ADP-ribose) polymerase, desmethylselegiline, trientine, and lipoic acid in transgenic ALS mice. Exp. Neurol. 2001;168:419-424; Khalili M, Eghtesadi S, Mirshafiey A, Eskandari G, Sanoobar M, Sahraian MA, Motevalian A, Norouzi A, Moftakhar S, Azimi A. Effect of lipoic acid consumption on oxidative stress among multiple sclerosis patients: a randomized controlled clinical trial. Nutr Neurosci. 2014;17:16-20; Skibska B, Goraca A. The protective effect of lipoic acid on selected cardiovascular diseases caused by age-related oxidative stress. Oxid. Med. Cell. Longev. 2015, art. no. 313021; Ghibu S, Richard C, Vergely C, Zeller M, Cottin Y, Rochette L. Antioxidant properties of an endogenous thiol: Alpha-lipoic acid, useful in the prevention of cardiovascular diseases. J. Card. Pharm. 2009;54:391-398; Rochette L, Ghibu S, Muresan A, Vergely C. Alpha-lipoic acid: Molecular mechanisms and therapeutic potential in diabetes. Can. J. Physiol. Phaarm. 2015;93:1021-1027). Furthermore, has been demonstrated that lipoic acid inhibits the initiation and promotion stages of carcinogenesis, suggesting it potential use as a chemopreventive agent (Novotny L, Rauko P, Cojocel C. alpha-Lipoic acid: the potential for use in cancer therapy. Neoplasma. 2008;55:81-6).

Are known boron containing compounds being enzyme inhibitors, such as hepatitis C virus serine protease inhibitors, β-lactamase, or boron containing compounds being anti-HIV agents and for boron neutron capture therapy (Bolt HM, Duydu Y, Ba 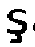 aran N, Golka K. Boron and its compounds: current biological research activities. Archiv. Toxicol. 2017; 1-4). Few natural products containing boron are easily accessible as starting materials for the design of new molecules and furthermore, only recently were discovered new synthetic pathways and new catalysts for introducing boron in organic molecules (Soriano-Ursúa MA, Das, BC, Trujillo-Ferrara, JG. Boron-containing compounds: Chemico-biological properties and expanding medicinal potential in prevention, diagnosis and therapy. Exp. Opin. Ther. Pat. 2014; 24:485-500).

Only two boronated compounds approved by the Food and Drug Administration (FDA) are known. The p-borophenyalanine is used in boron neutron capture therapy due to its ability to be transported inside the cancer cells by the transporter of L-amino acids, and it is safe for humans at doses up to 900 mg/kg. It is a drug for the treatment of malignant melanoma (Garabalino MA, Monti Hughes A, Molinari AJ, Heber EM, Pozzi ECC, Cardoso JE, Colombo LL, Nievas S, Nigg DW, Aromando RF, Itoiz ME, Trivillin VA, Schwint AE. Boron neutron capture therapy (BNCT) for the treatment of liver metastases: Biodistribution studies of boron compounds in an experimental model. Rad. Environ. Biophys. 2011; 50:199-207.; Nakamura H, Kirihata M. Boron compounds: New candidates for boron carriers in BNCT (2012) Neutron Capture Therapy: Principles and Applications, 9783642313349, pp. 99-116).

It is also known Bortezomib (Velcade®, Janssen-Cilag International N.V. as anticancer drug for the treatment of multiple myeloma.

Further boron compounds undergoing to clinical trials are known, such as dipeptidyl-peptidase IV (DPP4) inhibitors, anti-infective, antipsoriatic, and anti-cancer agents (Smoum R, Rubinstein A, Dembitsky VM, Srebnik M. Boron containing compounds as protease inhibitors. Chem. Rev. 2012; 112: 4156-4220). Further reference is made to US2001/039292.

### Technical problem

There is strong felt need in the pharmaceutical chemistry field, for new therapeutic boron compounds, which do not have the drawbacks of the compounds known in the art, in particular regarding toxicity and the limitations imposed on their dosage.

The inventors of the present invention have designed and synthesized new boronated compounds, structurally different from those known in the art, which have surprisingly proved to be effective and improved as neuroprotective and antiplatelet agents for the treatment of neurodegenerative, cardiovascular diseases and diabetes related to oxidative stress. Said compounds do not present toxicity and limitation in the dosage of the compounds known in the art. Furthermore, said compounds can simultaneously affect multiple target sites involved in the above reported diseases. In the field of neurodegenerative disorders, these compounds, in addition to the antioxidant properties of lipoic acid, show the ability of inhibiting the enzymes involved in the metabolism of catecholamines, whose deficiency characterizes diseases such as Alzheimer's disease or Parkinson's disease, and interfere with the deposition of protein aggregates. In the field of cardiovascular diseases, said compounds in addition to antioxidant activity, possess antiplatelet activity superior to lipoic acid, ensuring on the one hand the blocking of oxidative stress and limiting the progressions of lesions in blood vessels and on other the prevention of cardiovascular events without interfering with the physiological functions performed by platelets.

Moreover, some of the synthesized compounds show better physical-chemical properties, such as solubility and lipophilia compared to lipoic acid, to guarantee a better gastro-intestinal absorption and improved ability to cross biological barriers.

### Object of the invention

With reference to the attached claims, the technical problem is therefore solved by providing

### Compounds of formula (I):

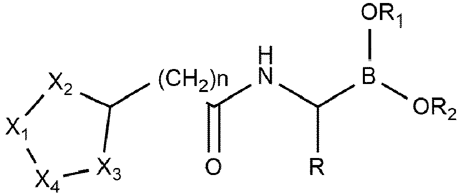

wherein
X₁ e X₂, the same or different, are CH, CH₂
the bond between X₁ e X₂ may be saturated or unsaturated,
X₃ and X₄ are the same and are S
n is an integer comprised between 1 and 10
R is H, alkyl selected from the group consisting of: methyl, ethyl, n-propyl, n-butyl, n-pentyl, n-hexyl, sec-butyl optionally substituted with a group selected from the group consisting of: adamantyl, i-propyl, sec-butyl, i-butyl, tert-butyl, i-pentyl, hydroxymethyl, 1-hydroxyethyl, sulfanylmethyl, 2- (methylsulfanyl)ethyl, carboxymethyl, carboxyethyl, 4-aminobutyl, 2-carbamoylethyl, 2-carbamoylmethyl, 2-methyl-3H-imidazole, 3-methyl-1H-indole, alkenyl selected from the group consisting of: ethenyl, 1-propenyl, 2-propenyl, 1-butenyl, 2-butenyl, 3-butenyl, isopentenyl, geranyl, farnesyl, optionally substituted with a group selected from the group consisting of: ethenyl, 2-propenyl, isopentenyl, geranyl, farnesyl, aryl optionally substituted with a group selected from the group consisting of: benzyl, 4-hydroxybenzyl, 3-alkyl-1H-indole, 2-methyl-1H-imidazole, 3,4-dihydroxyphenyl-methyl, 4-(2-aminoethyl)benzene-1,2-diol, 4-hydroxy-3methyl-phenylmethyl, 4-hydroxy-3-isopropyl-phenylmethyl, 3,4-dihydroxy-phenyl-ethyl, (3,4-dihydroxy-phenyl)-2-hydroxyethyl, (5-hydroxy-1H-indol-3-yl)ethyl, cyclic compounds having one or more cycles, consisting of a number of carbon atoms p comprised between 1 and 10, optionally substituted,
R₁ e R₂, the same or different, are H, CH₃, alkyl, alkenyl, aryl or R₁ and R₂ in a cyclic compound having one or more cycles, consisting of a number of carbon atoms m comprised between 1 and 10, optionally substituted,
their stereoisomers, enantiomers, conformers and racemic mixture of different stereoisomers.

And pharmaceutical composition comprising a pharmaceutical active amount of compound of formula (I) and suitable pharmaceutically acceptable excipients.

Further objects of the present invention are:
the compounds of formula (I) for use as a medicament,
the compounds of formula (I) for use as neuroprotector,
the compounds of formula (I) for use as antiplatelet,
the compounds of formula (I) for use as chemopreventive,
the compounds of formula (I) for use as antitumor,
the compounds of formula (I) for use as antimicrobial,
the compounds of formula (I) for use as antifungal,
the compounds of formula (I) for use in the treatment of oxidative stress,
the compounds of formula (I) for use in the treatment of neurodegenerative diseases related to oxidative stress,
the compounds of formula (I) for use in the treatment of cardiovascular diseases related to oxidative stress,
Further features of the present invention will be evident from the following detailed description with reference to the figures and experimental examples.

### Brief description of figures

Figure 1 shows in graph the results of in vitro evaluation of cytotoxicity of Aβ₁₋₄₂ peptide at different concentrations (0, 1.25, 2.5, 5, 10, 20, 40, 80, e 160 µM) in differentiated SH-SY5Y human neuroblastoma cells.
Figure 2 shows in graph the results of in vitro evaluation of neuroprotective effects of BLA, MEM, and LA on differentiated SH-SY5Y human neuroblastoma cells treated with Aβ₁₋₄₂ peptide for 24 hours, by MTT assay .
Figure 3 shows in graph the results of in vitro evaluation of neuroprotective effects of BLA, MEM, and LA in differentiated SH-SY5Y human neuroblastoma cells treated with Aβ₁₋₄₂ peptide by LDH assay.
Figure 4 shows in graph the results of in vitro evaluation of effects on AChE activity of BLA, MEM, and LA on of differentiated SH-SY5Y human neuroblastoma cells treated with BLA, MEM (25 and 50 µM) or medium only for 24 hours.
Figure 5. shows in graph the results of in vitro evaluation of TOS levels induced by BLA, MEM, and LA, in experimental model of Alzheimer's Disease.
Figure 6 shows in graph the results of in vitro evaluation of TOS levels induced BLA, MEM, and LA, in experimental model of Alzheimer's Disease.
Figure 7 shows in graph the results of vitality of PHWB cells after 24 h exposure to different BLA concentrations (0.1-100 µM); results are expressed as percentage of the control group (n = 6), data are expressed ad the mean ± SD. ** p < 0.05, * p < 0.1.
Figure 8 shows in graph the results of extracellular LDH levels in cultured PHWB cells in the presence of different BLA concentrations. ** p < 0.05, * p < 0.1.
Figure 9 shows in graph the results of vitality of PHWB cells after 24 h exposure to different LA concentrations; results are expressed as percentage of the control group (n = 6), data are expressed ad the mean ± SD. ** p < 0.05, * p < 0.1.
Figure 10 shows in graph the results of extracellular LDH levels in cultured PHWB cells in the presence of different LA concentrations. ** p < 0.05, * p < 0.1.
Figure 11. shows in graph the results of vitality of PHWB cells after 24 h exposure to different MEM concentrations;
results are expressed as percentage of the control group (n = 6), data are expressed ad the mean ± SD. ** p < 0.05, * p < 0.1.
Figure 12 shows in graph the results of extracellular LDH levels in cultured PHWB cells maintained in the presence of different MEM concentrations. ** p < 0.05, * p < 0.1.
Figure 13 shows in graph the results of effects of BLA, MEM, and LA treatments on sister chromatid exchange (SCE) rates in cultured human lymphocyte cells.
Figure 14 shows in graph the results of the presence of 8-oxo-2-deoxyguanosine (8-OH-dG) adducts in human blood cultures in the presence of different concentrations of BLA, MEM, and LA.
Figure 15 shows in graph the results of cytotoxicity of BLA and MEM at different concentrations on SH-SY5Y tumor cell line, by MTT assay.
Figure 16 shows in graph the results of cytotoxicity of BLA and MEM at different concentrations on SH-SY5Y tumor cell line, by LDH assay.

### Detailed description of the invention

### Definitions

Within the meaning of the present invention conformers refer to stereoisomers that are obtained by the rotation of a single bond in the molecule, giving rise to different conformations of the molecule.

Within the meaning of the present invention oxidative stress refers to the pathological condition characterized by the loss of physiological balance between the production and elimination of oxidizing chemical species and the alteration of the redox cellular environment with the production of peroxides and free radicals.

Within the meaning of the present invention pathologies related to oxidative stress refer to pathological conditions related to oxidative stress such as: neurodegenerative diseases, cardiovascular diseases, and diabetes.

Within the meaning of the present invention neurodegenerative diseases related to oxidative stress are Parkinson's disease, Alzheimer's disease, amyotrophic lateral sclerosis, multiple sclerosis, Huntington's disease, progressive supranuclear paralysis, frontotemporal dementia, Lewy's body dementia, Creutzfeldt-Jakob (MCJ), and Gerstmann-Straussler-Scheinker disease (GSS).

Within the meaning of the present invention cardiovascular diseases related to oxidative stress refer to atherosclerosis, hypertension, reperfusion ischemia, heart failure, and diabetic vasculopathy.

Within the meaning of the present invention carcinogenesis refers to the process that leads to the transformation of normal cell cells into cancer cells and the onset of the tumor.

Within the meaning of the present invention neuroprotector refers to an active ingredient or drug capable of slowing or stopping the progression of neuronal degeneration.

Within the meaning of the present invention chemopreventive refers to an active ingredient or drug capable of interrupting or regressing the carcinogenic process.

Within the meaning of the present invention antitumor refers to an active ingredient or drug capable of limiting the formation and spread of tumors.

Within the meaning of the present invention a platelet antiaggregant is an active ingredient or drug capable of negatively interacting with the function of platelet aggregation, thus preventing the formation of clots, thrombi, and emboli.

Within the meaning of the present invention antimicrobial refers to an active ingredient having bactericidal, fungicidal, virocidal, bacteriostatic, fungistatic and virostatic activity that can be used as antiseptics and disinfectants.

Within the meaning of the present invention pharmacologically effective amount refers to the dosage of the active ingredient which produces a therapeutic response or a desired effect without side effects.

Within the meaning of the present invention the pharmaceutically acceptable excipients are any chemical substance together with the active ingredient of a drug, for example, and not only, long-term stabilizing agents, bulking agents, filling (fillers), diluents, agents that facilitate drug absorption, viscosity-reducing agents, solubility-enhancing agents, agents that facilitate powder fluidity, agents that promote in-vitro stability, agents that prevent denaturing or aggregation, agents that improve the shelf-life; all also depending on the route of administration and dosage, which can be selected by the person skilled in the field considering of his common general knowledge.

The present invention provides the compounds of formula (I): 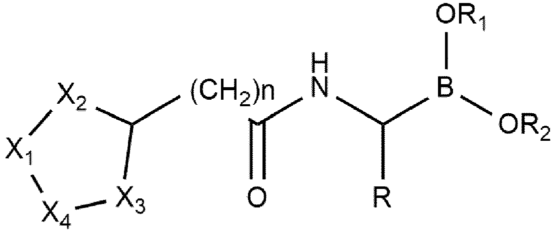 wherein
X₁ e X₂, the same or different, are CH, CH₂ the bond between X₁ e X₂ may be saturated or unsaturated,
X₃ and X₄ are the same and are S
n is an integer comprised between 1 and 10
R is H, alkyl selected from the group consisting of: methyl, ethyl, n-propyl, n-butyl, n-pentyl, n-hexyl, sec-butyl optionally substituted with a group selected from the group consisting of: adamantyl, i-propyl, sec-butyl, i-butyl, tert-butyl, i-pentyl, hydroxymethyl, 1-hydroxyethyl, sulfanylmethyl, 2- (methylsulfanyl)ethyl, carboxymethyl, carboxyethyl, 4-aminobutyl, 2-carbamoylethyl, 2-carbamoylmethyl, 2-methyl-3H-imidazole, 3-methyl-1H-indole, alkenyl selected from the group consisting of: ethenyl, 1-propenyl, 2-propenyl, 1-butenyl, 2-butenyl, 3-butenyl, isopentenyl, geranyl, farnesyl, optionally substituted with a group selected from the group consisting of: ethenyl, 2-propenyl, isopentenyl, geranyl, farnesyl, aryl optionally substituted with a group selected from the group consisting of: benzyl, 4-hydroxybenzyl, 3-alkyl-1H-indole, 2-methyl-1H-imidazole, 3,4-dihydroxyphenyl-methyl, 4-(2-aminoethyl)benzene-1,2-diol, 4-hydroxy-3methyl-phenylmethyl, 4-hydroxy-3-isopropyl-phenylmethyl, 3,4-dihydroxy-phenyl-ethyl, (3,4-dihydroxy-phenyl)-2-hydroxyethyl, (5-hydroxy-1H-indol-3-yl)ethyl, cyclic compounds having one or more cycles, consisting of a number of carbon atoms p comprised between 1 and 10, optionally substituted,
R₁ e R₂, the same or different, are H, CH₃, alkyl, alkenyl, aryl or R₁ and R₂ in a cyclic compound having one or more cycles, consisting of a number of carbon atoms m comprised between 1 and 10, optionally substituted,
their stereoisomers, enantiomers, conformers and racemic mixture of different stereoisomers.

Another object of the present invention is a pharmaceutical composition comprising a pharmacologically effective amount of the compounds of formula (I) 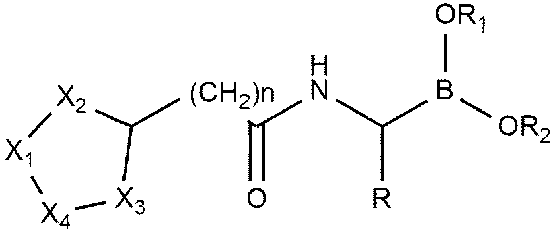 wherein
X₁ e X₂, the same or different, are CH, CH₂
the bond between X₁ e X₂ may be saturated or unsaturated,
X₃ and X₄ are the same and are S
n is an integer comprised between 1 and 10
R is H, alkyl selected from the group consisting of: methyl, ethyl, n-propyl, n-butyl, n-pentyl, n-hexyl, sec-butyl optionally substituted with a group selected from the group consisting of: adamantyl, i-propyl, sec-butyl, i-butyl, tert-butyl, i-pentyl, hydroxymethyl, 1-hydroxyethyl, sulfanylmethyl, 2- (methylsulfanyl)ethyl, carboxymethyl, carboxyethyl, 4-aminobutyl, 2-carbamoylethyl, 2-carbamoylmethyl, 2-methyl-3H-imidazole, 3-methyl-1H-indole, alkenyl selected from the group consisting of: ethenyl, 1-propenyl, 2-propenyl, 1-butenyl, 2-butenyl, 3-butenyl, isopentenyl, geranyl, farnesyl, optionally substituted with a group selected from the group consisting of: ethenyl, 2-propenyl, isopentenyl, geranyl, farnesyl, aryl optionally substituted with a group selected from the group consisting of: benzyl, 4-hydroxybenzyl, 3-alkyl-1H-indole, 2-methyl-1H-imidazole, 3,4-dihydroxyphenyl-methyl, 4-(2-aminoethyl)benzene-1,2-diol, 4-hydroxy-3methyl-phenylmethyl, 4-hydroxy-3-isopropyl-phenylmethyl, 3,4-dihydroxy-phenyl-ethyl, (3,4-dihydroxy-phenyl)-2-hydroxyethyl, (5-hydroxy-1H-indol-3-yl)ethyl, cyclic compounds having one or more cycles, consisting of a number of carbon atoms p comprised between 1 and 10, optionally substituted,
R₁ e R₂, the same or different, are H, CH₃, alkyl, alkenyl, aryl or R₁ and R₂ in a cyclic compound having one or more cycles, consisting of a number of carbon atoms m comprised between 1 and 10, optionally substituted,
their stereoisomers, enantiomers, conformers and racemic mixture of different stereoisomers.

Preferably X₁-X₂ is CH₂-CH₂, CH=CH.

More preferably X₁-X₂ is CH₂-CH₂.

Preferably, n is equal to 4.

Preferably R is H.

Preferably compounds of formula (I) are selected from the group consisting of:
5-((S)-1,2-dithiolan-3-yl)-*N*-(((3aR)-3a,5,5-trimethylhexahydro-4,6-methanebenzo[d][1,3,2,]dioxaborol-2-yl)methyl) pentanamide
(S)-((5-(1,2-dithiolan-3-yl)pentanamide)methyl)boronic acid
((S)-1-(5-((R)-1,2-dithiolan-3-yl)pentanamide)ethyl) boronic acid
((S)-1-(5-((R)-1,2-dithiolan-3-yl)pentanamide)-3-methyllbutyl) boronic acid
((S)-1-(5-((R)-1,2-dithiolan-3-yl)pentanamide)-2-mercaptoethyl) boronic acid
((1S)-1-(5-(1,2-dithiolan-3-yl)pentanamide)-2-(3,4-dihydroxyphenyl)ethyl) boronic acid
((S)-1-(5-((R)-1,2-dithiolan-3-yl)pentanamide)-2-(4-hydroxy-3-methylphenyl)ethyl) boronic acid
((S)-1-(5-((R)-1,2-dithiolan-3-yl)pentanamide)-2-(4-hydroxy-3-isopropylphenyl)ethyl) boronic acid
((S)-1-(5-((R)-1,2-dithiolan-3-yl)pentanamide)-3-(3,4-dihydorxyphenyl)propyl) boronic acid
((3S)-1-(5-((R)-1,2-dithiolan-3-yl)pentanamide)-3-(3,4-dihydroxyphenyl)-3-hydroxypropyl) boronic acid
((S)-1-(5-((R)-1,2-dithiolan-3-yl)pentanamide)-2-hydroxyethyl) boronic acid
(1-(5-((R)-1,2-dithiolan-3-yl)pentanamide)-4-methylpent-3-en-1-yl) boronic acid
((S)-1-(5-((R)-1,2-dithiolan-3-yl)pentanamide)-3-(5-hydroxy-1H-indol-3-yl)propyl) boronic acid
5-((R)-1,2-dithiolan-3-yl)-N-((1S)-1-(3a,5,5-trimethylhexahydro-1H-4,6-methanebenzo[c]borol-2(3H)-yl)etyl)pentanamide
5-((R)-1,2-dithiolan-3-yl)-N-((1S)-3-methyl-1-(3a,5,5-trimethylhexahydro-4,6- methanebenzo [d][1,3,2]dioxaborol-2-yl)butyl)pentanamide
N-((1S)-2-(3,4-dihydroxyphenyl)-1-(3a,5,5- trimethylhexahydro-4,6-methanebenzo[d][1,3,2]dioxaborol-2-yl)etyl)-5-((R)-1,2-dithiolan-3-yl)pentanamide
5-((R)-1,2-dithiolan-3-yl)-N-((1S)-2-mercapto-1-(3a,5,5-trimethylhexahydro-4,6-methanebenzo[d][1,3,2]dioxaborol-2-yl)etyl)pentanamide
5-((R)-1,2-dithiolan-3-yl)-N-((1S)-2-(4-hydroxy-3-isopropylphenyl)-1-(3a,5,5- trimethylhexahydro -4,6-metanebenzo[d][1,3,2]dioxaborol-2-yl)etyl)pentanamide 5-((R)-1,2-dithiolan-3-yl)-N-((1S)-2-(4-hydroxy-3-methylphenyl)-1-(3a,5,5-trimethylhexahydro-4,6-metanebenzo[d][1,3,2]dioxaborol-2-yl)etyl)pentanamide N-((3S)-3-(3,4-dihydroxyphenyl)-3-hydroxy-1-(3a,5,5-trimethylhexahydro-4,6-metanebenzo[d][1,3,2]dioxaborol-2-yl)propyl)-5-((R)-1,2-dithiolan-3-yl)pentanamide N-((1S)-3-(3,4-dihydroxyphenyl)-1-(3a,5,5- trimethylhexahydro-4,6-metanebenzo[d][1,3,2]dioxaborol-2-yl)propyl)-5-((R)-1,2-ditiolan-3-yl)pentanamide
5-((R)-1,2-dithiolan-3-yl)-N-((1S)-2-hydroxy-1-(3a,5,5-trimethylhexahydro-4,6-metanebenzo[d][1,3,2]dioxaborol-2-yl)etyl)pentanamide
5-((R)-1,2-dithiolan-3-yl)-N-((1S)-3-(5-hydroxy-1H-indol-3-yl)-1-(3a,5,5- trimethylhexahydro-4,6-metanobenzo[d][1,3,2]dioxaborol-2-yl)propyl)pentanamide 5-((R)-1,2-dithiolan-3-yl)-N-(4-methyl-1-(3a,5,5-trimethylhexahydro-4,6-metanebenzo[d][1,3,2]dioxaborol-2-yl)pent-3-en-1-yl)pentanamide

Further object of the present invention are the compounds of formula (I) for use as a medicament.

Further object of the present invention are the compounds of formula (I) for use as neuroprotector.

Further object of the present invention are the compounds of formula (I) for use as antiplatelet.

Further object of the present invention are the compounds of formula (I) for use as chemopreventive.

Further object of the present invention are the compounds of formula (I) for use as antitumor.

Further object of the present invention are the compounds of formula (I) for use as antimicrobial.

Further object of the present invention are the compounds of formula (I) for use as antifungal.

Further object of the present invention are the compounds of formula (I) for use in the treatment of oxidative stress.

Further object of the present invention are the compounds of formula (I) for use in the treatment of neurodegenerative diseases related to oxidative stress.

Further object of the present invention are the compounds of formula (I) for use in the treatment of cardiovascular diseases related to oxidative stress.

Preferably neurodegenerative diseases related to oxidative stress are selected from the group consisting of: Parkinson's disease, Alzheimer's disease, amyotrophic lateral sclerosis, multiple sclerosis, Huntington's disease, progressive supranuclear paralysis, frontotemporal dementia, Lewy's body dementia, Creutzfeldt-Jakob (MCJ), and Gerstmann-Straussler-Scheinker disease (GSS).

Preferably cardiovascular diseases related to oxidative stress are selected from the group consisting of: atherosclerosis, hypertension, reperfusion ischemia, heart failure, diabetic vasculopathy.

The compounds of the present invention have proved to be non-cytotoxic and non-genotoxic even at high concentrations. Notably, their effect on neurodegenerative diseases occurs because they are able to reduce cell death induced by toxic compounds, such as Aβ-peptides, and inhibit the enzymes involved in the degradation of catecholamines.

### Examples

Materials: All the reagents, unless otherwise stated, were from Sigma Aldrich Co. (St. Louis, MO, USA). All other chemicals used were of the highest purity commercially available. Chromatographic purifications were performed on silica gel using column chromatography (Merck 60, 70-230 mesh ASTM silica gel), and compounds were detected with UV light (λ = 254 nm). Before performing biological studies, chemical structures and purities (> 98%) of boronated compounds were confirmed by ¹H-, ¹³C-NMR, MS spectra, and HPLC analysis. NMR spectra were recorded with a Varian VXR-300 spectrometer (300 MHz). MS spectra were recorded using Mass Spectrometer (Thermo Finnigan LCQ Deca XP Plus) and a Phenomenex C18 150 x 2.1 mm, 5 µm column. The capillary temperature was set at 300 °C. The purity of the boronated compounds was determined by analytical HPLC using a Waters 600 HPLC equipped with a X-Bridge BEH130 C-18, 5 µm, 4.6 x 250 mm column with Waters 2996 PDA detector, and H₂O/CH₃CN (0.1% TFA) as solvent system in the form of a linear gradient from 10 to 90% of CH₃CN over 70 min and a flow rate of 1 mL/min. SH-SY5Y (CRL-2266™) cell line was provided from ATCC (Rockville, MD, USA). Amyloid β protein fragments 1-42 were purchased from Sigma (Saint Louis, USA) and penicillin/streptomycin (10,000 U/mL) from Thermo Fisher® (Waltham, MA USA). Dulbecco's Modified Eagle's Medium/Nutrient Mixture F-12 Ham (1:1 mixture), Fetal Bovine Serum (FBS), Trypsin-EDTA solution, All-trans-Retinoic acid, and BDNF (Brain Derived Neurotrophic Factor, Sigma Aldrich®) were also provided from Sigma Aldrich®. ADP and Collagen for platelet aggregation were purchased from Hart Biologicals (Hartlepool, UK) .

### Synthesis of 5-((S)-1,2-dithiolan-3-yl)-N-(((3aR)-3a,5,5-trimethylhexahydro-4,6-methanobenzo[d] [1,3,2]dioxaborol-2-yl)methyl)pentanamide (BLA)

To a stirred solution of aminoboronate·HCl (**1**) (550 mg, 2.11 mmol) (Gorovoy AS, Gozhina OV, Svendsen JS, Domorad AA, Tetz GV, Tetz VV, Lejon T. Boron-containing peptidomimetics - A novel class of selective anti-tubercular drugs. Chem. Biol. Drug Des. 2013, 81:408-413) in dry dichloromethane (4 mL) triethylamine (0.73 mL, 5.27 mmol) and then LA (435 mg, 2.11 mmol) were added at room temperature under stirring. After 10 minutes, Benzotriazol-1-yloxy)tris(dimethylamino)phosphonium hexafluorophosphate (BOP) (1.212 g, 2.74 mmol) in dry dichloromethane (2 mL) was added. After 3.5 h at room temperature, the reaction mixture was removed under vacuum. The residue was taken up in dichloromethane and washed with KHSO₄ 1N, NaHCO₃, and brine; the organic layer was dried over anhydrous Na₂SO₄ and the solvent was removed under vacuum. Chromatography was performed on silica gel with CH₂Cl₂:AcOEt (9:1) as eluant to give 376 mg of 5-((S)-1,2-dithiolan-3-yl)-N-(((3aR)-3a,5,5-trimethylhexahydro-4,6-methanobenzo[d] [1,3,2]dioxaborol-2-yl)methyl)pentanamide (BLA) . Yield: 44.8%; *R*_{f} = 0.23, CHCl₃; ¹H-NMR (CDCl₃) δ: 0.77 (3H, s), 0.79 (3H, s), 1.25 (2H, m), 1.29 (3H, s), 1.42 (1H, m), 1.47 (2H, m), 1.49 (CH, m), 1.65 (2H, m), 1.97 (2H, m), 2.01 (2H, m), 2.10 (2H, m), 2.31 (2H, m), 2.48 (2H, m), 3.01 (2H, m), 3.51 (CH, m), 4.35 (CH), 7.15 (1H, NH); ¹³C-NMR (CDCl₃) δ: 23.95, 24.58, 26.29, 26.43, 26.96, 28.42, 28.76, 34.58, 35.59, 38.33, 39.33, 40.20, 50.98, 51.38, 56.31, 78.46, 84.73, 86.96, 179.69; MS (ESI) m/z 398.19 [M + H]⁺.

### Synthesis of (S)-((5-(1,2-dithiolan-3-yl)-pentanamido)methyl)boronic acid (BLA1)

Phenylboronic acid (273 mg, 2.24 mmol) was added to a solution of BLA (230 mg, 0.56 mmol) in diethyl ether/water (1:1) (14 mL) and the reaction mixture was stirred for 12h. The aqueous phase was filtered, washed with diethyl ether and concentrated under vacuum to give the pure product (quantitative yield). Yield: 30%. ¹H-NMR (d₆-DMSO) δ: 1.25 (2H, m), 1.53 (2H, m), 1.57 (2H, m), 1.99 (2H, m), 2.21 (2H, m), 2.53 (1H, m), 2.60 (2H, m), 3.29 (2H, m), 7.37 (1H, NH); ¹³C-NMR (CDCl₃) δ: 25.83, 27.61, 34.79, 36.59, 38.40, 39.83, 47.20, 56.31, 173.69; MS (ESI) m/z 264.08 [M + H]⁺.

Cell cultures and differentiation: SH-SY5Y cell line were growth in DMEM:F12 (1:1) media including 10% FBS and 1% penicillin/streptomycin at 37 °C and 5% CO₂ until cells reach confluence. Then, cells harvested via using trypsin/EDTA solution and transferred to 48 well plates. For differentiation, media were changed to supplemented media with 10 µg/mL retinoic acid and incubated for 5 days. Finally, media were changed into serum free media supplementing with both 10 µM retinoic acid and 25 ng/mL BDNF. Differentiated cells were determined under invert microscope and the cells were used within 10 days (Encinas M, Iglesias M, Liu Y, Wang H, Muhaisen A, Ceña V, Gallego C, Comella JX. Sequential treatment of SH-SY5Y cells with retinoic acid and brain-derived neurotrophic factor gives rise to fully differentiated, neurotrophic factor-dependent, human neuron-like cells. J. Neurochem. 2002; 75:991-1003 e Cheung YT, Lau WKW, Yu MS, Lai CSW, Yeung SC, So KF, Chang RCC. Effects of all-trans-retinoic acid on human SH-SY5Y neuroblastoma as in vitro model in neurotoxicity research. Neurotoxicol. 2009; 30:127-135).

LDH assay: For LDH assay application, LDH cytotoxicity assay kit (Cayman Chemical Company®, Ann Arbor, MI, USA) was used according to the manufacturer's recommendations. The cells were transferred to 48-well plates and different concentrations of drugs were applied for 24 h. After that, 100 µL supernatant and 100 µL of reaction mixture were transferred to a fresh 48-well plate and incubated for 30 min at room temperature. Finally, the absorbance of the cultures was analyzed at 490 nm using a microplate reader as reported by Turkez H. Togar B, Di Stefano A, Taspinar N, Sozio P. Protective effects of cyclosativene on H2O2-induced injury in cultered rat primary cerebral cortx cells. Cytotechnol. 2015; 67:299-309.

Determination of AChE activity: the activity of AChE within the cellular Alzheimer Disease's model was measured by the Acetylcholinesterase Assay Kit (Colorimetric) from Abcam^{(R)} (Cambridge, MA, USA) according to the manufacturer's recommended protocol.

TAC and TOS analysis: the automated TAC and TOS assays were carried out by commercially available kits (Rel Assay^{(R)} Diagnostics, Gaziantep, Turkey) on samples from the cultures.

Apoptosis detection by Hoechst 33258 staining: Hoechst 33258 staining was used to detect apoptotic nuclei. The positive control (only Aβ₁₋₄₂, 20 µM), negative control and BLA/MEM (25 and 50 µM) with β-amyloid cultures were incubated for 24 h to analyze cell morphology. Then, cells fixed with 4% paraformaldehyde in phosphate buffered saline at 4 °C for 30 min. After washing cells with PBS, the nuclear DNAs were incubated with 1 mM Hoechst 33258 fluorescent dye for 5 min at room temperature. Cells were observed and photographed under a fluorescence microscopy (Leica® DM IL LED).

Apoptosis-Necrosis assay: the frequencies of viable, apoptotic and necrotic cells were detected with the Annexin V-FITC apoptosis detection Kit I (BD Pharmingen, USA). Briefly, 5 x 10⁴ cells were collected by centrifugation and cells resuspended in 500 µL of 1X binding buffer. Then, 5 µL of Annexin V-FITC and 5 µl of propidium iodide (PI 50 µg/ml) were added to the cultures and incubated 5 min in the dark. After that, cells fixed with 4% paraformaldehyde in phosphate buffered saline at 4 °C for 30 min. Cells from each well were stained according to the manufacturer's instructions and analyzed using a flow cytometer (CyFlow Cube 6, Partec, Germany). A computer system was used for data acquisition and analysis. Four different populations of cells are detected with the Annexin V-FITC kit: viable cells that are annexin negative and PI negative and express no fluorescence, early apoptotic cells that are annexin positive and PI negative and that express green fluorescence, late apoptotic/necrotic cells that are annexin positive and PI positive and that express green and orange fluorescence, necrotic cells that are annexin negative and PI positive and that express orange fluorescence (Majstorovic I, Vucevic D, Pavlovic B, Vasilijic S, Colic M. An anti-DEC-205 monoclonal antibody stimulates binding of thymocytes to rat thymic dendritic cells and promotes apoptosis of thymocytes. Cent Eur J Immunol. 2014; 39:411-418) .

Toxicological evaluation: for evaluating biosafety of formulations, we used cultured human peripheral blood cells. The blood cultures were set up according to a slight modification of the protocol described by Evans and O'Riordan (Human peripheral blood lymphocytes for the analysis of chromosome aberrations in mutagen tests. Mutat Res. 1975; 31:135-148). Human blood samples were obtained from six healthy volunteers, non-smoking, non-alcoholic, not under drug therapy and with no recent history of exposure to mutagens; females aged 28-30 years. All volunteers have signed informed consent. The heparinized blood (0.5 mL⁻¹) was cultured in 6.0 mL⁻¹ of culture medium (PB-MAX) Karyotyping Medium Gibco, Spain) with 5.0 mg/mL⁻¹ of phytohemagglutinin (Sigma Aldrich^{(R)}, Steinheim, Germany). Different concentrations of BLA, LA, and MEM were added to the cultures just before the incubation. For assessing cytotoxicity and genotoxicity potentials of the drugs, MTT, LDH, Sister chromatid exchange (SCE) and 8-hydroxy-2'-deoxyguanosine assays were performed.

SCE assay: to provide successive visualization of SCEs, 5-bromo-2-deoxyuridine was added at culture cells. Exactly 70 h and 30 min after beginning of the incubations, demecolcine (N-Diacetyl-N-methyl colchicine) was added to the cultures. After hypotonic treatment (0.075 M KCl), followed by three repetitive cycles of fixation in methanol/acetic acid solution (3:1, v/v), centrifugation and re-suspension, the cell suspension was dropped onto chilled, grease-free microscopic slides, air-dried, aged for three days, and then differentially stained for the inspection of the SCE rate according to the fluorescence plus Giemsa (FPG) procedure. For each treatment condition, well-spread twenty-five second division metaphases containing 42-46 chromosomes per cell were scored and the values obtained were calculated as SCEs per cell.

Nucleic acid oxidation: 8-hydroxy-2'-deoxyguanosine assay kits (Cayman Chemical®) was used for determining 8- OH-dG levels in the cultures. It is a competitive assay that can be used for the quantification of 8-OHdG in homogenates and recognizes both free 8-OHdG and DNA-incorporated 8-OH-dG. This assay depends on the competition between 8-OH-dG and 8-OH-dGacetylcholinesterase (AChE) conjugate for a limited amount of 8-OH-dG monoclonal antibody (Abdel-Wahab BA, Metwally ME. Ginkgo biloba enhances the anticonvulsant and neuroprotective effects of sodium valproate against kainic acid-induced seizures in mice. J Pharmacol Toxicol. 2011; 6:679-690). All procedures were carried out in accordance with the provider's manual.

Blood Collection and Preparation of Platelet-Rich Plasma: blood was collected from healthy and non-smoking volunteers (all volunteers have signed informed consent) into a polypropylene tube containing 0.105 M buffered sodium citrate. None of the donors had received any medication known to affect platelet function for at least 10 days before blood collection. Platelet-rich plasma (PRP) was collected by the centrifugation of whole blood at 150 g for 15 min at room temperature. Subsequently, platelet-poor plasma (PPP) was collected by the centrifugation at 2000 g for 20 min at room temperature. The platelet count was adjusted to 2x10⁸ cells /ml with PPP.

Incubation of PRP with BLA and LA: PRP was incubated with BLA and LA (25, 50, and 100 µM) for 15 minutes at 37 °C. PRP without any compound was used as a control.

Optical Aggregometry in Platelet-Rich Plasma: the measurement of platelet aggregation in PRP was performed using four-channel aggregometer (APACT 4004; LABiTec, Ahrensburg, Germany). After the incubation of PRP with three different compounds, platelet aggregation was stimulated by ADP (20 µM) and Collagen (10 µg/mL) and percentage of aggregation was monitored for 10 minutes. The percentage of aggregation, slope and shape change parameters were monitored for 10 minutes. Assay was started 1 hour after blood was collected and finished within 3 hours (Hao HZ, He AD, Wang DC, Yin Z, Zhou YJ, Liu G, Liang ML, Da XW, Yao GQ, Xie W, Xiang JZ, Ming ZY. Antiplatelet activity of loureirin A by attenuating Akt phosphorylation: In vitro studies. Eur. J. Pharmacol. 2015; 746:63-9).

Antimicrobial activity: antimicrobial activity of BLA was determined using agar disc diffusion method (Bauer AW, Kirby WM, Sherris JC, Turck M. Antibiotic susceptibility testing by standardized single disc method. Am J Clin Pathol. 1966; 45:493-496). For this purpose, two Gram-positive (*Staphylococcus aureus* ATCC 25923 and *Enterococcus faecalis* ATCC 29212) and two Gram-negative (*Escherichia coli* ATCC 25922 and *Pseudomonas aeruginosa* ATCC 27853) strains, and one fungal strain (*Candida albicans* ATCC 90028) were chosen based on their clinical and pharmacological importance. The bacterial and fungal stock cultures were incubated for overnight at 37 °C on Mueller-Hinton Agar (MHA) and Sabourad 2% Dextrose Agar (SDA) medium, respectively. BLA was dissolved in dimethylsulfoxide (DMSO), sterilized by filtration using sintered glass filter, and stored at 4 °C. MHA and SDA plates were seeded with indicator bacterial and fungal strains and allowed to stay at 37 °C for 3 h. Blank discs were saturated with 5 µL of BLA solution (0.15 µg/disc). Disc that saturated by DMSO were prepared as negative control and discs contain different standards like ampicillin, imipenem and tetracycline were used as positive control. Then, saturated discs were directly loaded on the cultured MHA and SDA plate in aseptic conditions. The zones of growth inhibition around the discs were measured after 24 hours of in incubation at 37 °C for bacteria and 48 hours for fungi at 37 °C.

Statistical analysis: the results were expressed by mean ± S.D. from at least six independent experiments. For statistical comparisons, quantitative data was analysed by one-way analysis of variance (ANOVA) followed by Duncan's test according to the statistical program SPSS software (version 20.0, SPSS, Chicago, IL, USA). A p-value less than 0.05 was considered as significant.

Treatments: cells at 70-80% confluence were treated with concentrations of BLA, MEM, LA (0.1, 1, 10, 25, 50, and 100 µM) and Aβ₁₋₄₂ peptide (20 µM) for 24 h. Control cells (n = 6) were cultured under normal conditions. 1% Triton X, ascorbic acid (10 µM) and hydrogen peroxide (H₂O₂, 25 µM) were used as positive controls for cell viability, TAC and TOS analysis, respectively.

MTT assay: according to the manufacturer's instructions (Cayman Chemical Company®, Ann Arbor, MI, USA), 3-(4,5-Dimethylthiazol-2-yl)-2,5-diphenyltetrazolium bromide (MTT) solution was applied to cell cultures. As summary, MTT was added and incubated in the cell cultures for 3 h and after incubation dimethyl sulfoxide (DMSO) was used to solve formazan crystals, and a plate reader was used to analyse cultures at 570 nm wavelength (Togar B, Turkez H, Hacimuftuoglu A, Tatar A, Geyikoglu F. Guaiazulene biochemical activity and cytotoxic and genotoxic effects on rat neuron and N2a neuroblastom cells. J. Intercult. Ethnopharmacol. 2015; 4:29-33).

### Example 1. Synthesis

Syntheses of boronated compounds were performed using several coupling reagents. Removal of protecting groups was achieved using mild conditions. All compounds were purified using chromatographic techniques and chemically characterized using nuclear magnetic resonance (NMR) and mass spectrometry (MS) instruments.

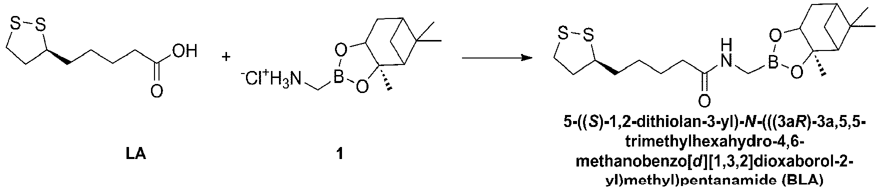

Reagents and conditions: (Benzotriazol-1-yloxy)tris(dimethylamino)phosphonium hexafluorophosphate, triethylamine, dichloromethane, 3.5 h, room temperature.

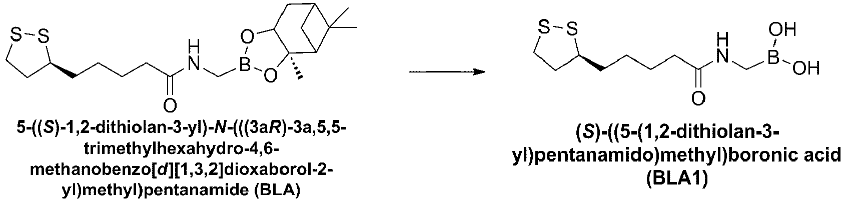

Reagents and conditions: phenylboronic acid, diethyl ether/water, room temperature.

### Example 2. Effects of Aβ₁₋₄₂ peptide treatments on the viability of differentiated SH-SY5Y cells

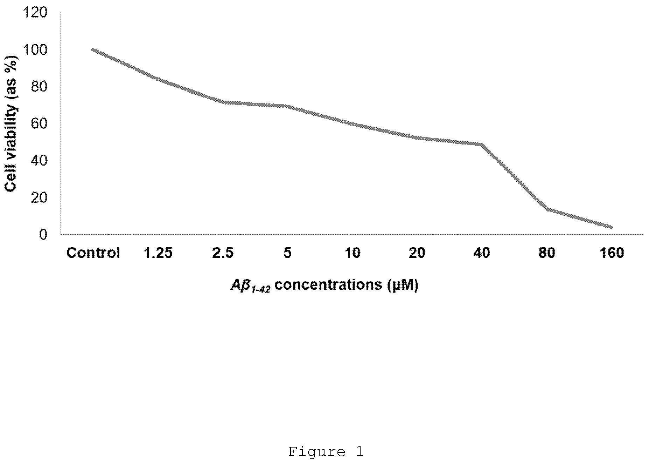

SH-SY5Y human neuroblastoma cells were differentiated to a neuronal-like state *in vitro* using a combination of retinoic acid (RA) and brain-derived neurotrophic factor (BDNF). For establishing the effective concentration of *Aβ*_{*1*-*42*} fragments on the viability of differentiated SH-SY5Y cells as an experimental model for Alzheimer's, a wide range of *Aβ₁₋₄₂* concentrations (0, 1.25, 2.5, 5, 10, 20, 40, 80, and 160 µM) were added into the cell cultures for 24 h. SH-SY5Y cells with differentiation were exposed to *Aβ₁₋₄₂* for 24 h and then assessed for cell viability by MTT assay. The results of MTT analysis indicated that *Aβ* toxicity was in a clear concentrations dependent manner. 52.1% and 48% of the SH-SY5Y cells showed decreased levels of cell viability after the 24h treatment with 20 and 40µM *Aβ*_{*1*-*42*} (Figure 1). The protocol using the 24 h treatment of 20 µM *Aβ₁₋₄₂* was selected for further experiments of this investigation.

### EXAMPLE 3. Effects of BLA, MEM and LA on Aβ₁-₄₂-induced death of differentiated SH-SY5Y cells

3-(4,5-dimethylthiazol-2-yl)-2,5-diphenyltetrazolium bromide (MTT) and lactate dehydrogenase (LDH) assays were used to evaluate the neuroprotective effects of 5-((S)-1,2-dithiolan-3-yl)-N-(((3aR)-3a,5,5-trimethylhexahydro-4,6-methanobenzo[d][1,3,2]dioxaborol-2-yl)methyl)pentanamide (BLA), memantine (MEM), and lipoic acid (LA) against *Aβ₁₋₄₂* cytotoxicity. The cultures without BLA, MEM, or LA were studied as negative control group. 1% Triton-X was added to the culture media to provide negative control group. MTT reduction and LDH release observed after 24 h of *Aβ*_{*1*-*42*}-exposure at 20 µM. Cell viability rates after *Aβ*_{*1*-*42*}-exposure were found as 52.1% and 42.6%, in MTT and LDH assays, respectively. When BLA, MEM, or LA (0.1 - 100 µM) was added to the cultured cells with 20 µM *Aβ₁₋₄₂* both BLA, MEM, LA raised the number of viable cells significantly more than cells treated with *Aβ₁₋₄₂* only (P < 0.01).

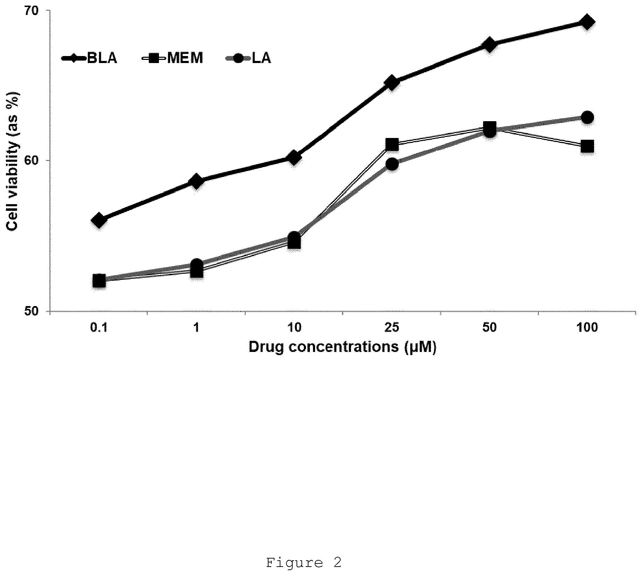

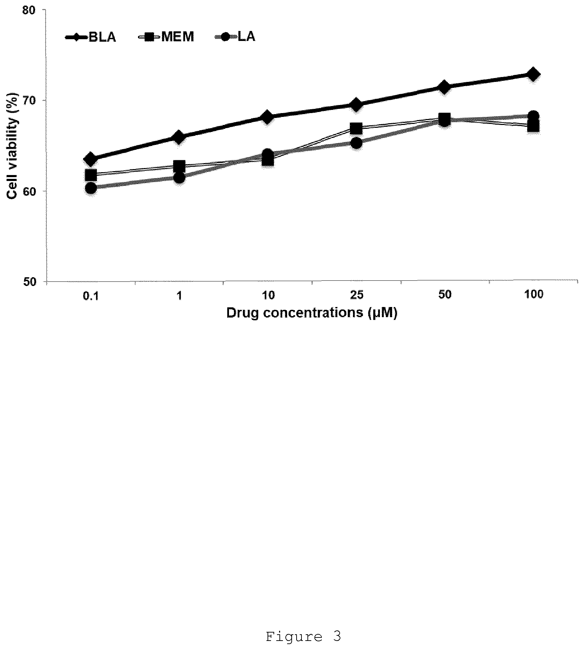

MEM significantly inhibited cell death induced by *Aβ*_{*1*-*42*}-exposure, resulting in an increase in the cell viability by 17.27 %, 19.38%, 17.08% at concentrations of 25, 50, and 100 µM, respectively (MTT assay). LA significantly inhibited cell death induced by *Aβ*_{*1*-}*₄₂-*exposure, resulting in an increase in the cell viability by 14.78 %, 19.01%, 20.73% at concentrations of 25, 50, 100 µM, respectively (MTT assay). However, BLA significantly inhibited cell death induced by *Aβ*_{*1*-}*₄₂-*exposure, resulting in an increase in the cell viability by 25.14 %, 29.94%, 32.92% at concentrations of 25, 50, 100 µM, respectively (MTT assay) (Figure 2). Likewise, MEM significantly inhibited LDH release induced by *Aβ*_{*1*-}*₄₂-*exposure. resulting in an increase in the cell viability by 56.81%, 59.15%, 57.28% at concentrations of 25, 50, 100 µM, respectively. LA significantly inhibited LDH release induced by *Aβ*_{*1*-}*₄₂-*exposure, resulting in an increase in the cell viability by 53.05%, 58.68%, 59.86% at concentrations of 25, 50, 100 µM, respectively. However, BLA significantly inhibited LDH release induced by *Aβ*_{*1*-}*₄₂-*exposure, resulting in an increase in the cell viability by 62.91%, 67.37%, 70.65% at concentrations of 25, 50, 100 µM, respectively. These results suggested that BLA was more effective than LA and MEM in alleviating *Aβ*_{*1*-}*₄₂-*induced cell death (Figure 3).

### Example 4. Effects of BLA and MEM on acetylcholinesterase (AChE) activity in cellular model of Alzheimer's disease.

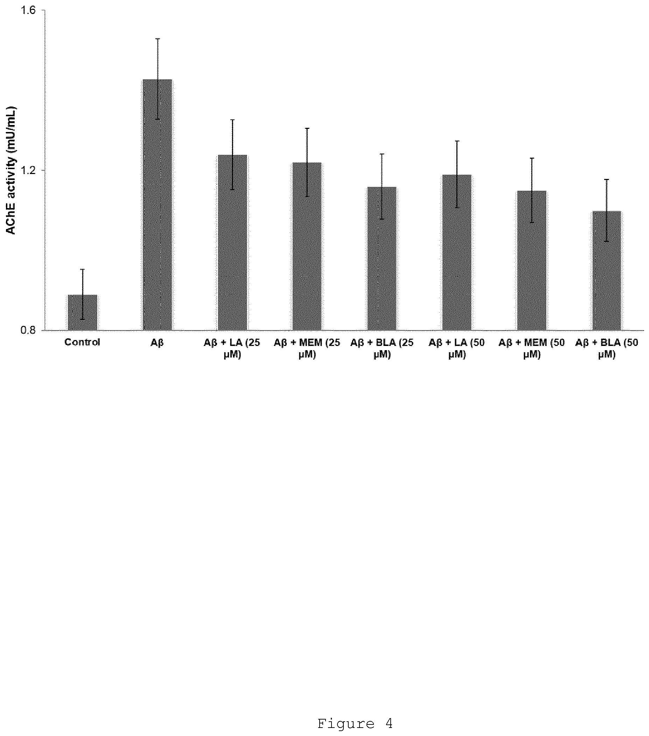

AChE activity was evaluated in differentiated SH-SY5Y cells treated with BLA, LA, MEM (25 and 50 µM) or media alone for 24 h. At 20 µM concentration of *Aβ*_{*1*-}*₄₂,* AChE activity caused an approximate 60% increase in baseline. On the other hand, at 25 and 50 µM concentrations of BLA, MEM and LA reduced *Aβ*-induced AChE activity in rates of 18.77%, 22.96%; 14.57%, 19.47% and 13.17%, 16.66%, respectively (Figure 4) suggesting that BLA was more effective than LA and MEM in reducing AChE activity.

### EXAMPLE 5. Effects of BLA and MEM on Total Antioxidant Capacity (TAC) and Total Oxidative Status (TOS) levels in cellular model of Alzheimer's disease.

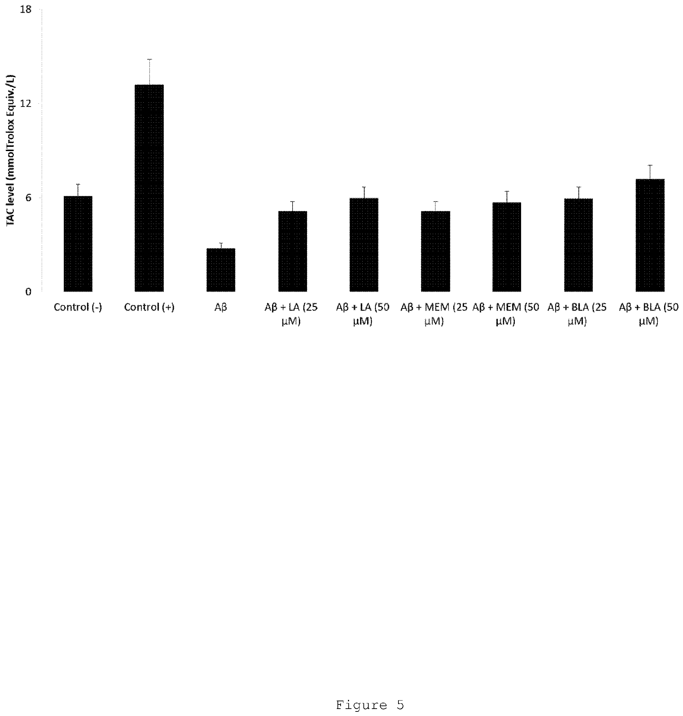

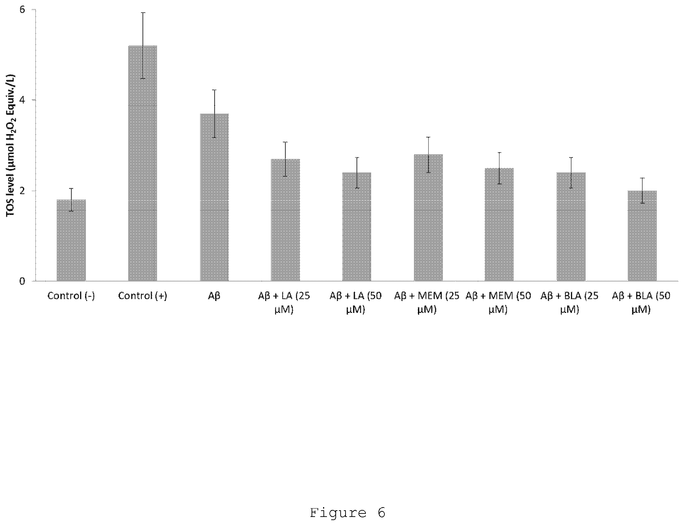

Figure 5 and 6 show the effects of BLA and MEM treatments on TAC and TOS levels in cellular model of Alzheimer's disease, respectively. The obtained results revealed that *Aβ₁₋₄₂* exposure caused significant (*P* < 0.0.1) decreases of TAC and increases of TOS levels *in vitro.* On the contrary, these alterations were modulated by BLA, MEM, or LA co-treatments with *Aβ*_{*1*-}*₄₂.* BLA was found as more effective than MEM and LA in alleviating oxidative stress by *Aβ*_{*1*-}*₄₂-*exposure.

### EXAMPLE 6. Effects of BLA and MEM on apoptosis and necrosis in cellular model of Alzheimer's disease.

Staining with Hoechst 33258 revealed that a co-treatment with BLA, LA, or MEM (25 and 50 µM) protected nuclear integrity and inhibited apoptosis by *Aβ*_{*1*-}*₄₂.* Likewise, the observations by using apoptosis-necrosis assay indicated that Aβ toxicity occurred via a necrotic rather than an apoptotic pathway. Data demonstrated that BLA, LA, or MEM protected from *Aβ*_{*1*-}*₄₂-*induced necrosis in a dose-dependent manner.

Flow cytometric analysis of apoptosis and necrosis showed that *Aβ₁₋₄₂* application caused a significant (P<0.05) cell death rate (66.09%) via necrosis. On the contrary, BLA, LA, and MEM decreased the necrotic cell percentage by *Aβ₁₋₄₂* in the rates of %68.38, %61.67, and %65.97, respectively. Apoptosis was not observed in flow cytometric investigations and so, it could be concluded that *Aβ*_{*1*-*42*} caused cell death through necrotic pathways. BLA was considered as more able to protect the human neuron-like cells from *Aβ*_{*1*-}*₄₂-*induced necrosis and late apoptosis than LA or MEM.

### Example 7. Cytotoxicity assay

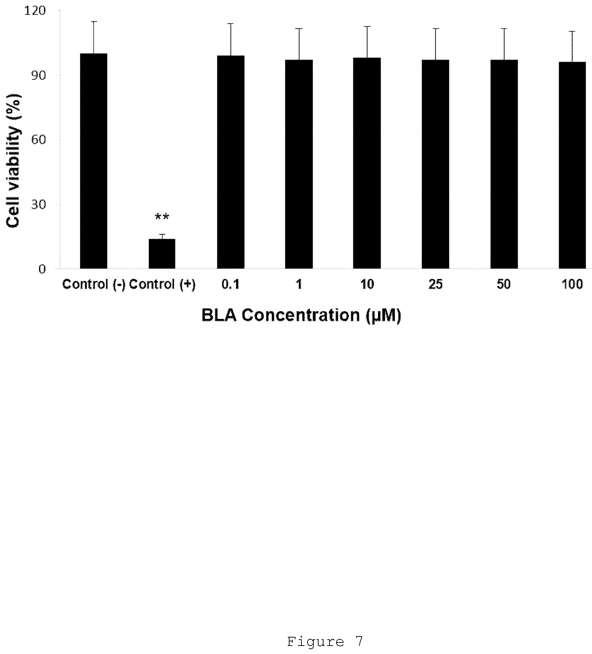

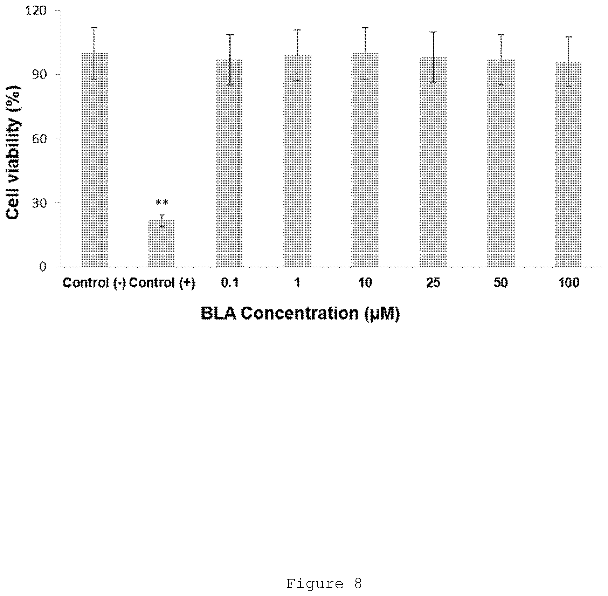

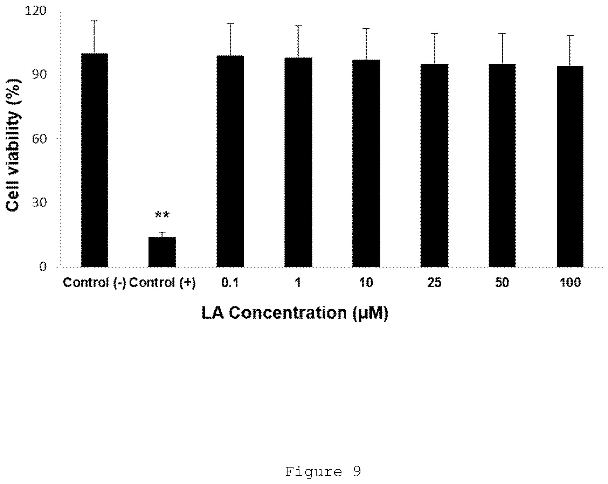

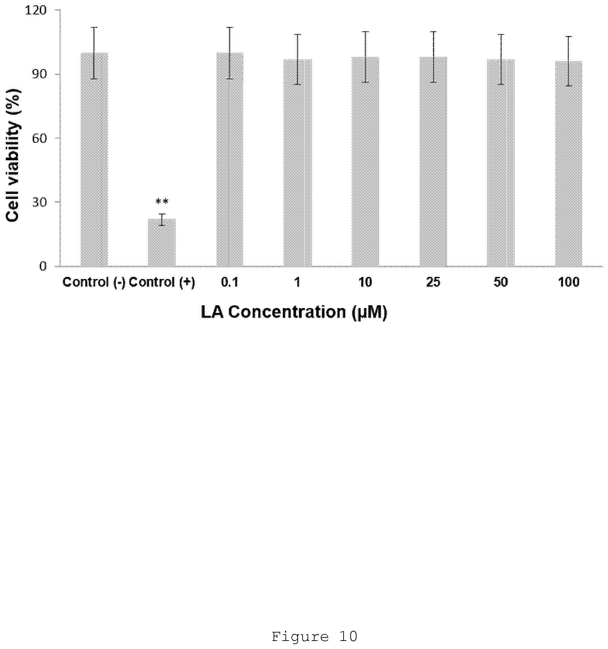

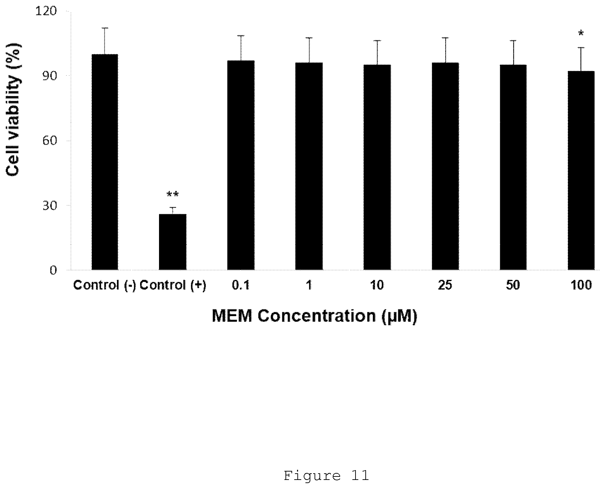

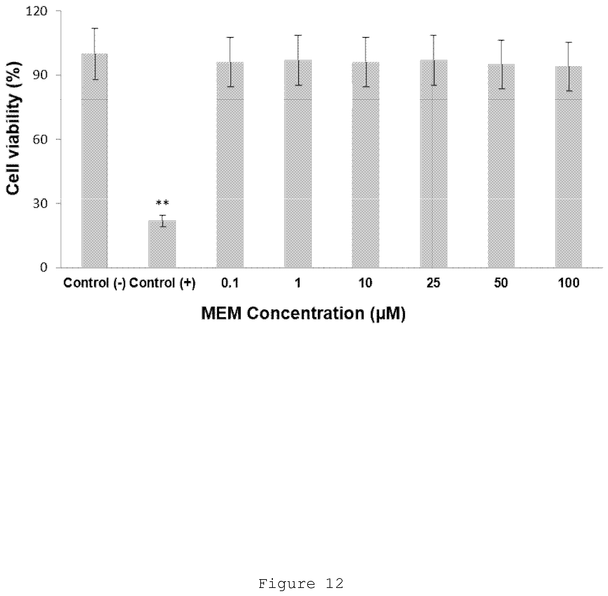

MTT and LDH assays were performed to measure cell death in response to different concentrations of BLA, MEM, and LA. The cultured peripheral human whole blood (PHWB) cells were exposed to 0.1 to 100 µM of BLA, MEM and LA. Both BLA and LA did not show any significant changes in cell viability during 24 h, as determined by MTT and LDH assays (Figures 7-10). Likewise, the results of both cytotoxicity assays showed that up to 50 µM of MEM did not cause any significant changes in cell viability, but the application with 100 µM of MEM caused a slight toxicity (p > 0.05, p < 0.1, MTT assay) compared to the untreated group (Figures 11 and 12).

### EXAMPLE 8. Genotoxicity/oxidative DNA damage assay.

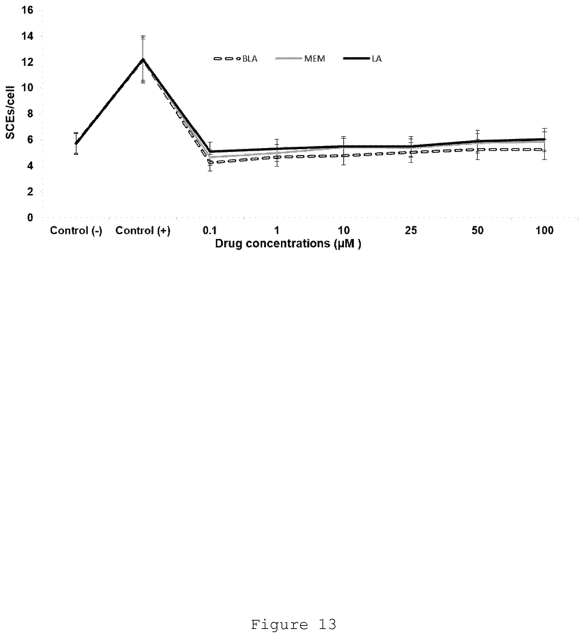

The frequencies of sister chromatid exchanges (SCEs) with increasing BLA concentrations were investigated in cultured human lymphocytes. As shown in Figure 13, there were not significant differences in the frequencies of SCEs between the control group and all BLA treated groups (P > 0.05). Similarly, the levels of SCEs in MEM and LA treated groups were not higher than the control group (P > 0.05) (Figure 13).

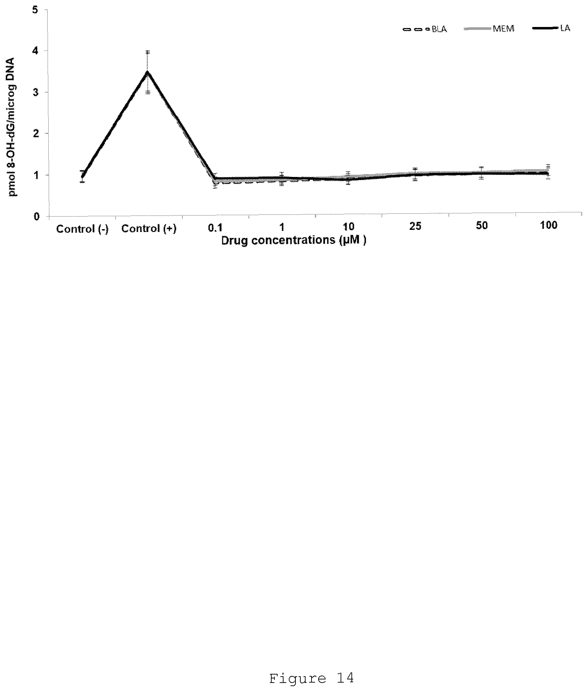

The status of 8-OH-dG in cultured human blood cells of the control and BLA, MEM, and LA treated groups is reported in Figure 14. The levels of 8-OH-dG, a sensitive marker of oxidative DNA damage, were quantified about mitomycin (MMC) treatment. Results showed that MMC (at 10⁻⁷ M) significantly increased 8-OH-dG concentrations in human blood cultures after 24 h. On the contrary, 8-OH-dG levels were not increased in the blood cells that were treated with BLA, MEM, and LA concentrations.

### EXAMPLE 9. Anti-platelet activity.

The biological evaluations revealed that all tested concentrations of BLA possessed strong activity of anti-platelet aggregation induced by ADP and collagen (Tables 1 and 2) .

**Table 1**

| Groups | Max. Aggregation (%) | Slope (%) | Shape change (%) |
|---|---|---|---|
| Control | 77.5 | 82.6 | 28.8 |
| BLA (25 µM) | 49,8* | 66.7* | 12.1* |
| BLA (50 µM) | 41.3* | 51.5* | 10.3* |
| BLA (100 µM) | 32.2* | 47.3* | 6.8* |
| LA (25 µM) | 55.8* | 69.8* | 17.4* |
| LA (50 µM) | 47.8* | 61.4* | 14.5* |
| LA (100 µM) | 44.6* | 58.0* | 13.8* |

| | | | |
|---|---|---|---|
| *p < 0.01 | | | |

**Table 2**

| Groups | Max. Aggregation (%) | Slope (%) | Shape change (%) |
|---|---|---|---|
| Control | 74.7 | 72.0 | 47.9 |
| BLA (25 µM) | 45.7* | 41.4* | 24.7* |
| BLA (50 µM) | 31.8* | 30.8* | 10.2* |
| BLA (100 µM) | 7.9* | 25.5* | 5.6* |
| LA (25 µM) | 47.9* | 43.6* | 27.9* |
| LA (50 µM) | 41.6* | 37.2* | 23.4* |
| LA (100 µM) | 35.8* | 31,4* | 20.8* |

| | | | |
|---|---|---|---|
| *p < 0.01 | | | |

### EXAMPLE 10. Antitumor activity of BLA and MEM

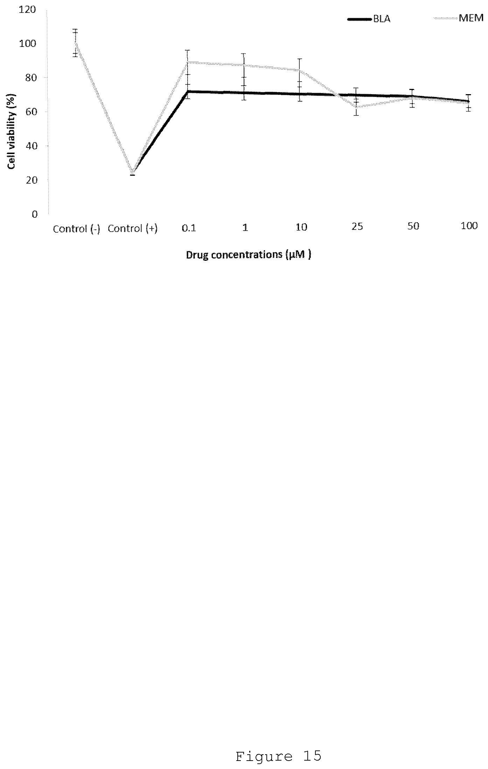

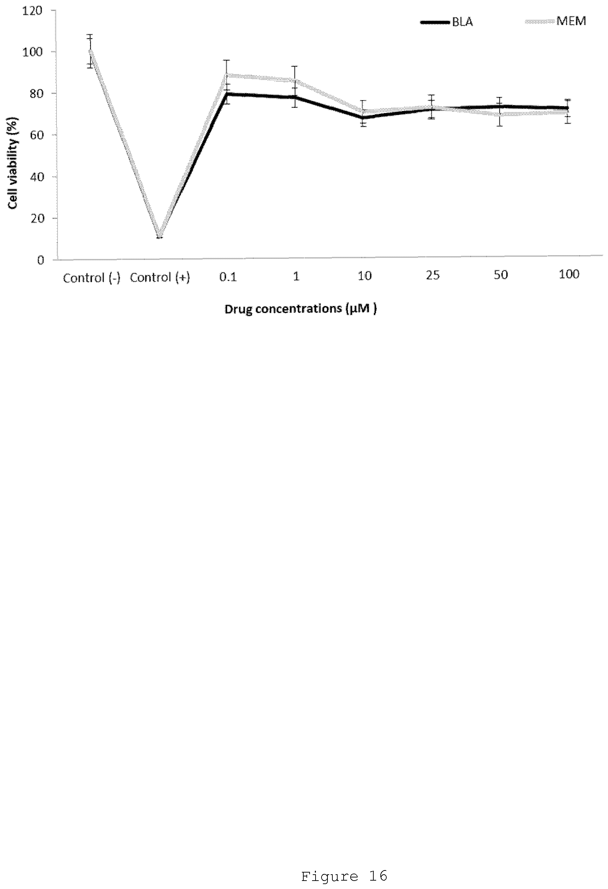

Antitumor activity of BLA and MEM was tested on SHSY-5Y human neuroblastoma cell line using MTT and LDH assays. Cells were grown to near-confluency before treatment. Growth of the cell line was inhibited by both drug application in a dose-dependent manner. BLA and MEM showed moderate antitumor effects. However, the lower concentrations (< 50 µM) of BLA was found more effective than MEM. Results proved that the activity of BLA against tumor cells was greater than MEM (Figures 15 and 16).

### Example 11. Antimicrobial activity of BLA.

Antimicrobial activity of BLA was determined using agar disc diffusion method (Bauer AW, Kirby WM, Sherris JC, Turck M. Antibiotic susceptibility testing by standardized single disc method. Am J Clin Pathol. 1966; 45:493-496). Two Gram-positive (*Staphylococcus aureus* ATCC 25923 and *Enterococcus faecalis* ATCC 29212) and two Gram-negative (*Escherichia coli* ATCC 25922 and *Pseudomonas aeruginosa* ATCC 27853) strains, and one fungal strain (*Candida albicans* ATCC 90028) were chosen based on their clinical and pharmacological importance. Results showed that BLA possess modest antimicrobial activity against the tested strains (Table 3).

**Table 3. Antimicrobial activity (MIC µg/mL)**

| Compound | *S. aureus* ATCC 25923 | *E. faecalis* ATCC 29212 | *E. coli* ATCC 25922 | *P*. *aeruginosa* ATCC 27853 | *C*. *albicans* ATCC 90028 |
|---|---|---|---|---|---|
| BLA | 256 | 256 | 128 | 256 | 128 |

## Claims

1. Compounds of formula (I): 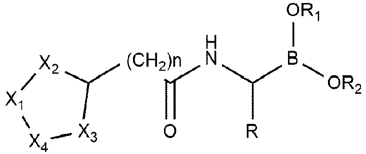 Wherein
X₁ and X₂, the same or different, are CH, CH₂
the bond between X₁ and X₂ may be satured or unsatured
X₃ and X₄ are the same and are S
n is an integer comprised between 1 and 10
R is H, alkyl selected from the group consisting of: methyl, ethyl, n-propyl, n-butyl, n-pentyl, n-hexyl, sec-butyl optionally substituted with a group selected from the group consisting of: adamantyl, i-propyl, sec-butyl, i-butyl, tert-butyl, i-pentyl, hydroxymethyl, 1-hydroxyethyl, sulfanylmethyl, 2-(methylsulfanyl)ethyl, carboxymethyl, carboxyethyl, 4-aminobutyl, 2-carbamoylethyl, 2-carbamoylmethyl, 2-methyl-3H-imidazole, 3-methyl-1H-indole, alkenyl selected from the group consisting of: ethenyl, 1-propenyl, 2-propenyl, 1-butenyl, 2-butenyl, 3-butenyl, isopentenyl, geranyl, farnesyl optionally substituted with a group selected from the group consisting of: ethenyl, 2-propenyl, isopentenyl, geranyl, farnesyl, aryl optionally substituted with a group selected from the group consisting of: benzyl, 4-hydroxybenzyl, 3-alkyl-1H-indole, 2-methyl-1H-imidazole, 3,4-dihydroxyphenyl-methyl, 4-(2-aminoethyl)benzene-1,2-diol, 4-hydroxy-3methyl-phenylmethyl, 4-hydroxy-3-isopropyl-phenyl-methyl, 3,4-dihydroxy-phenyl-ethyl, (3,4-dihydroxy-phenyl)-2-hydroxyethyl, (5-hydroxy-1H-indol-3-yl)ethyl, cyclic compounds having one or more cycles, consisting of a number of carbon atoms p comprised between 1 and 10, optionally substituted,
R₁ and R₂, the same or different, are H, CH₃, alkyl, alkenyl, aryl or R1 are R₂ in a cyclic compound having one or more cycles, consisting of a number of carbon atoms m comprised between 1 and 10, optionally substituted,
their stereoisomers, enantiomers, conformers and racemic mixture of different stereoisomers.

2. Compounds of formula (I) according to claim 1 wherein X₁-X₂ is CH₂-CH₂, CH=CH.

3. Compounds of formula (I) according to claim 1 wherein n is 4.

4. Compounds of formula (I) according to claim 1 wherein R is H.

5. Compounds of formula (I) according to claim 1 selected from the group consisting of:
5-((S)-1,2-dithiolan-3-yl)-*N*-(((3aR)-3a,5,5-trimethylhexahydro-4,6-methanebenzo[d][1,3,2,]dioxaborol-2-yl)methyl) pentanamide
(*S*)-((5-(1,2-dithiolan-3-yl)pentanamide)methyl)boronic acid
((S)-1-(5-((R)-1,2-dithiolan-3-yl)pentanamide)ethyl) boronic acid ((S)-1-(5-((R)-1,2-dithiolan-3-yl)pentanamide)-3-methyllbutyl) boronic acid
((S)-1-(5-((R)-1,2-dithiolan-3-yl)pentanamide)-2-mercaptoethyl) boronic acid
((1S)-1-(5-(1,2-dithiolan-3-yl)pentanamide)-2-(3,4-dihydroxyphenyl)ethyl) boronic acid
((S)-1-(5-((R)-1,2-dithiolan-3-yl)pentanamide)-2-(4-hydroxy-3-methylphenyl)ethyl) boronic acid
((S)-1-(5-((R)-1,2-dithiolan-3-yl)pentanamide)-2-(4-hydroxy-3-isopropylphenyl)ethyl) boronic acid
((S)-1-(5-((R)-1,2-dithiolan-3-yl)pentanamide)-3-(3,4-dihydorxyphenyl)propyl) boronic acid
((3S)-1-(5-((R)-1,2-dithiolan-3-yl)pentanamide)-3-(3,4-dihydroxyphenyl)-3-hydroxypropyl) boronic acid
((S)-1-(5-((R)-1,2-dithiolan-3-yl)pentanamide)-2-hydroxyethyl) boronic acid
(1-(5-((R)-1,2-dithiolan-3-yl)pentanamide)-4-methylpent-3-en-1-yl) boronic acid
((S)-1-(5-((R)-1,2-dithiolan-3-yl)pentanamide)-3-(5-hydroxy-1H-indol-3-yl)propyl) boronic acid
5-((R)-1,2-dithiolan-3-yl)-N-((1S)-1-(3a,5,5-trimethylhexahydro-1H-4,6-methanebenzo[c]borol-2(3H)-yl)etyl)pentanamide
5-((R)-1,2-dithiolan-3-yl)-N-((1S)-3-methyl-1-(3a,5,5-trimethylhexahydro-4,6- methanebenzo [d][1,3,2]dioxaborol-2-yl)butyl)pentanamide
N-((1S)-2-(3,4-dihydroxyphenyl)-1-(3a,5,5- trimethylhexahydro-4,6-methanebenzo[d][1,3,2]dioxaborol-2-yl)etyl)-5-((R)-1,2-dithiolan-3-yl)pentanamide
5-((R)-1,2-dithiolan-3-yl)-N-((1S)-2-mercapto-1-(3a,5,5-trimethylhexahydro-4,6-methanebenzo[d] [1,3,2]dioxaborol-2-yl)etyl)pentanamide
5-((R)-1,2-dithiolan-3-yl)-N-((1S)-2-(4-hydroxy-3-isopropylphenyl)-1-(3a,5,5- trimethylhexahydro-4,6-metanebenzo[d][1,3,2]dioxaborol-2-yl)etyl)pentanamide
5-((R)-1,2-dithiolan-3-yl)-N-((1S)-2-(4-hydroxy-3-methylphenyl)-1-(3a,5,5-trimethylhexahydro-4,6-metanebenzo[d][1,3,2]dioxaborol-2-yl)etyl)pentanamide
N-((3S)-3-(3,4-dihydroxyphenyl)-3-hydroxy-1-(3a,5,5-trimethylhexahydro-4,6-metanebenzo[d] [1,3,2]dioxaborol-2-yl)propyl)-5-((R)-1,2-dithiolan-3-yl)pentanamide
N-((1S)-3-(3,4-dihydroxyphenyl)-1-(3a,5,5- trimethylhexahydro-4,6-metanebenzo[d][1,3,2]dioxaborol-2-yl)propyl)-5-((R)-1,2-ditiolan-3-yl)pentanamide
5-((R)-1,2-dithiolan-3-yl)-N-((1S)-2-hydroxy-1-(3a,5,5-trimethylhexahydro-4,6-metanebenzo[d][1,3,2]dioxaborol-2-yl)etyl)pentanamide
5-((R)-1,2-dithiolan-3-yl)-N-((1S)-3-(5-hydroxy-1H-indol-3-yl)-1-(3a,5,5-trimethylhexahydro-4,6-metanobenzo[d] [1,3,2]dioxaborol-2-yl)propyl)pentanamide
5-((R)-1,2-dithiolan-3-yl)-N-(4-methyl-1-(3a,5,5-trimethylhexahydro-4,6-metanebenzo[d] [1,3,2]dioxaborol-2-yl)pent-3-en-1-yl)pentanamide

6. Compounds of formula (I): 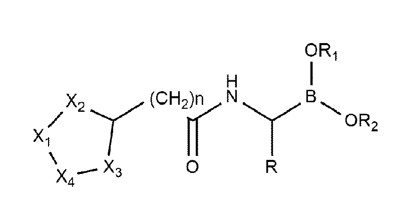 Wherein X₁ and X₂, the same or different, are CH, CH₂, the bond between X₁ and X₂ may be saturated or unsaturated, wherein X₃ and X₄ are the same and are S, n is an integer comprised between 1 and 10, R is H, alkyl selected from the group consisting of: methyl, ethyl, n-propyl, n-butyl, n-pentyl, n-hexyl, sec-butyl optionally substituted with a group selected from the group consisting of: adamantyl, i-propyl, sec-butyl, i-butyl, tert-butyl, i-pentyl, hydroxymethyl, 1-hydroxyethyl, sulfanylmethyl, 2-(methylsulfanyl)ethyl, carboxymethyl, carboxyethyl, 4-aminobutyl, 2-carbamoylethyl, 2-carbamoylmethyl, 2-methyl-3H-imidazole, 3-methyl-1H-indole, alkenyl selected from the group consisting of: ethenyl, 1-propenyl, 2-propenyl, 1-butenyl, 2-butenyl, 3-butenyl, isopentenyl, geranyl, farnesyl optionally substituted with a group selected from the group consisting of: ethenyl, 2-propenyl, isopentenyl, geranyl, farnesyl, aryl optionally substituted with a group selected from the group consisting of: benzyl, 4-hydroxybenzyl, 3-alkyl-1H-indole, 2-methyl-1H-imidazole, 3,4-dihydroxyphenyl-methyl, 4-(2-aminoethyl)benzene-1,2-diol, 4-hydroxy-3methyl-phenylmethyl, 4-hydroxy-3-isopropyl-phenylmethyl, 3,4-dihydroxy-phenyl-ethyl, (3,4-dihydroxy-phenyl)-2-hydroxyethyl, (5-hydroxy-1H-indol-3-yl)ethylcyclic compounds having one or more cycles, consisting of a number of carbon atoms p comprised between 1 and 10, optionally substituted, R₁ and R₂, the same or different, are H, CH₃, alkyl, alkenyl, aryl or R1 are R₂ in a cyclic compound having one or more cycles, consisting of a number of carbon atoms m comprised between 1 and 10, optionally substituted, their stereoisomers, enantiomers, conformers and racemic mixture of different stereoisomers, for use as a medicament.

7. Compounds of formula (I): 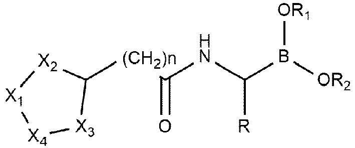 Wherein X₁ and X₂, the same or different, are CH, CH₂, the bond between X₁ and X₂ may be saturated or unsaturated, wherein X₃ and X₄ are the same and are S, n is an integer comprised between 1 and 10, R is H, alkyl selected from the group consisting of: methyl, ethyl, n-propyl, n-butyl, n-pentyl, n-hexyl, sec-butyl optionally substituted with a group selected from the group consisting of: adamantyl, i-propyl, sec-butyl, i-butyl, tert-butyl, i-pentyl, hydroxymethyl, 1-hydroxyethyl, sulfanylmethyl, 2-(methylsulfanyl)ethyl, carboxymethyl, carboxyethyl, 4-aminobutyl, 2-carbamoylethyl, 2-carbamoylmethyl, 2-methyl-3H-imidazole, 3-methyl-1H-indole, alkenyl selected from the group consisting of: ethenyl, 1-propenyl, 2-propenyl, 1-butenyl, 2-butenyl, 3-butenyl, isopentenyl, geranyl, farnesyl optionally substituted with a group selected from the group consisting of: ethenyl, 2-propenyl, isopentenyl, geranyl, farnesyl, aryl optionally substituted with a group selected from the group consisting of: benzyl, 4-hydroxybenzyl, 3-alkyl-1H-indole, 2-methyl-1H-imidazole, 3,4-dihydroxyphenyl-methyl, 4-(2-aminoethyl)benzene-1,2-diol, 4-hydroxy-3methyl-phenylmethyl, 4-hydroxy-3-isopropyl-phenylmethyl, 3,4-dihydroxy-phenyl-ethyl, (3,4-dihydroxy-phenyl)-2-hydroxyethyl, (5-hydroxy-1H-indol-3-yl)ethyl cyclic compounds having one or more cycles, consisting of a number of carbon atoms p comprised between 1 and 10, optionally substituted, R₁ and R₂, the same or different, are H, CH₃, alkyl, alkenyl, aryl or R1 are R₂ in a cyclic compound having one or more cycles, consisting of a number of carbon atoms m comprised between 1 and 10, optionally substituted, their stereoisomers, enantiomers, conformers and racemic mixture of different stereoisomers, for use as neuroprotector.

8. Compounds of formula (I): 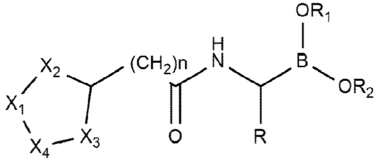 Wherein X₁ and X₂, the same or different, are CH, CH₂, the bond between X₁ and X₂ may be saturated or unsaturated, wherein X₃ and X₄ are the same and are S, n is an integer comprised between 1 and 10, R is H, alkyl selected from the group consisting of: methyl, ethyl, n-propyl, n-butyl, n-pentyl, n-hexyl, sec-butyl optionally substituted with a group selected from the group consisting of: adamantyl, i-propyl, sec-butyl, i-butyl, tert-butyl, i-pentyl, hydroxymethyl, 1-hydroxyethyl, sulfanylmethyl, 2-(methylsulfanyl)ethyl, carboxymethyl, carboxyethyl, 4-aminobutyl, 2-carbamoylethyl, 2-carbamoylmethyl, 2-methyl-3H-imidazole, 3-methyl-1H-indole, alkenyl selected from the group consisting of: ethenyl, 1-propenyl, 2-propenyl, 1-butenyl, 2-butenyl, 3-butenyl, isopentenyl, geranyl, farnesyl optionally substituted with a group selected from the group consisting of: ethenyl, 2-propenyl, isopentenyl, geranyl, farnesyl, aryl optionally substituted with a group selected from the group consisting of: benzyl, 4-hydroxybenzyl, 3-alkyl-1H-indole, 2-methyl-1H-imidazole, 3,4-dihydroxyphenyl-methyl, 4-(2-aminoethyl)benzene-1,2-diol, 4-hydroxy-3methyl-phenylmethyl, 4-hydroxy-3-isopropyl-phenylmethyl, 3,4-dihydroxy-phenyl-ethyl, (3,4-dihydroxy-phenyl)-2-hydroxyethyl, (5-hydroxy-1H-indol-3-yl)ethyl cyclic compounds having one or more cycles, consisting of a number of carbon atoms p comprised between 1 and 10, optionally substituted, R₁ and R₂, the same or different, are H, CH₃, alkyl, alkenyl, aryl or R1 are R₂ in a cyclic compound having one or more cycles, consisting of a number of carbon atoms m comprised between 1 and 10, optionally substituted, their stereoisomers, enantiomers, conformers and racemic mixture of different stereoisomers, for use as antiplatelet.

9. Compounds of formula (I): 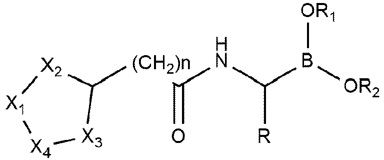 Wherein X₁ and X₂, the same or different, are CH, CH₂, the bond between X₁ and X₂ may be saturated or unsaturated, wherein X₃ and X₄ are the same and are S, n is an integer comprised between 1 and 10, R is H, alkyl selected from the group consisting of: methyl, ethyl, n-propyl, n-butyl, n-pentyl, n-hexyl, sec-butyl optionally substituted with a group selected from the group consisting of: adamantyl, i-propyl, sec-butyl, i-butyl, tert-butyl, i-pentyl, hydroxymethyl, 1-hydroxyethyl, sulfanylmethyl, 2-(methylsulfanyl)ethyl, carboxymethyl, carboxyethyl, 4-aminobutyl, 2-carbamoylethyl, 2-carbamoylmethyl, 2-methyl-3H-imidazole, 3-methyl-1H-indole, alkenyl selected from the group consisting of: ethenyl, 1-propenyl, 2-propenyl, 1-butenyl, 2-butenyl, 3-butenyl, isopentenyl, geranyl, farnesyl optionally substituted with a group selected from the group consisting of: ethenyl, 2-propenyl, isopentenyl, geranyl, farnesyl, aryl optionally substituted with a group selected from the group consisting of: benzyl, 4-hydroxybenzyl, 3-alkyl-1H-indole, 2-methyl-1H-imidazole, 3,4-dihydroxyphenyl-methyl, 4-(2-aminoethyl)benzene-1,2-diol, 4-hydroxy-3methyl-phenylmethyl, 4-hydroxy-3-isopropyl-phenylmethyl, 3,4-dihydroxy-phenyl-ethyl, (3,4-dihydroxy-phenyl)-2-hydroxyethyl, (5-hydroxy-1H-indol-3-yl)ethyl cyclic compounds having one or more cycles, consisting of a number of carbon atoms p comprised between 1 and 10, optionally substituted, R₁ and R₂, the same or different, are H, CH₃, alkyl, alkenyl, aryl or R1 are R₂ in a cyclic compound having one or more cycles, consisting of a number of carbon atoms m comprised between 1 and 10, optionally substituted, their stereoisomers, enantiomers, conformers and racemic mixture of different stereoisomers, for use as chemopreventive.

10. Compounds of formula (I): 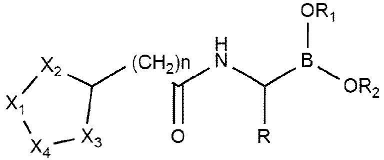 Wherein X₁ and X₂, the same or different, are CH, CH₂, the bond between X₁ and X₂ may be saturated or unsaturated, wherein X₃ and X₄ are the same and are S, n is an integer comprised between 1 and 10, R is H, alkyl selected from the group consisting of: methyl, ethyl, n-propyl, n-butyl, n-pentyl, n-hexyl, sec-butyl optionally substituted with a group selected from the group consisting of: adamantyl, i-propyl, sec-butyl, i-butyl, tert-butyl, i-pentyl, hydroxymethyl, 1-hydroxyethyl, sulfanylmethyl, 2-(methylsulfanyl)ethyl, carboxymethyl, carboxyethyl, 4-aminobutyl, 2-carbamoylethyl, 2-carbamoylmethyl, 2-methyl-3H-imidazole, 3-methyl-1H-indole, alkenyl selected from the group consisting of: ethenyl, 1-propenyl, 2-propenyl, 1-butenyl, 2-butenyl, 3-butenyl, isopentenyl, geranyl, farnesyl optionally substituted with a group selected from the group consisting of: ethenyl, 2-propenyl, isopentenyl, geranyl, farnesyl, aryl optionally substituted with a group selected from the group consisting of: benzyl, 4-hydroxybenzyl, 3-alkyl-1H-indole, 2-methyl-1H-imidazole, 3,4-dihydroxyphenyl-methyl, 4-(2-aminoethyl)benzene-1,2-diol, 4-hydroxy-3methyl-phenylmethyl, 4-hydroxy-3-isopropyl-phenyl-methyl, 3,4-dihydroxy-phenyl-ethyl, (3,4-dihydroxy-phenyl)-2-hydroxyethyl, (5-hydroxy-1H-indol-3-yl)ethyl cyclic compounds having one or more cycles, consisting of a number of carbon atoms p comprised between 1 and 10, optionally substituted, R₁ and R₂, the same or different, are H, CH₃, alkyl, alkenyl, aryl or R1 are R₂ in a cyclic compound having one or more cycles, consisting of a number of carbon atoms m comprised between 1 and 10, optionally substituted, their stereoisomers, enantiomers, conformers and racemic mixture of different stereoisomers, for use as antitumor.

11. Compounds of formula (I): 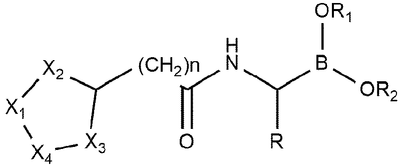 Wherein X₁ and X₂, the same or different, are CH, CH₂, the bond between X₁ and X₂ may be saturated or unsaturated, wherein X₃ and X₄ are the same and are S, n is an integer comprised between 1 and 10, R is H, alkyl selected from the group consisting of: methyl, ethyl, n-propyl, n-butyl, n-pentyl, n-hexyl, sec-butyl optionally substituted with a group selected from the group consisting of: adamantyl, i-propyl, sec-butyl, i-butyl, tert-butyl, i-pentyl, hydroxymethyl, 1-hydroxyethyl, sulfanylmethyl, 2-(methylsulfanyl)ethyl, carboxymethyl, carboxyethyl, 4-aminobutyl, 2-carbamoylethyl, 2-carbamoylmethyl, 2-methyl-3H-imidazole, 3-methyl-1H-indole, alkenyl selected from the group consisting of: ethenyl, 1-propenyl, 2-propenyl, 1-butenyl, 2-butenyl, 3-butenyl, isopentenyl, geranyl, farnesyl optionally substituted with a group selected from the group consisting of: ethenyl, 2-propenyl, isopentenyl, geranyl, farnesyl, aryl optionally substituted with a group selected from the group consisting of: benzyl, 4-hydroxybenzyl, 3-alkyl-1H-indole, 2-methyl-1H-imidazole, 3,4-dihydroxyphenyl-methyl, 4-(2-aminoethyl)benzene-1,2-diol, 4-hydroxy-3methyl-phenylmethyl, 4-hydroxy-3-isopropyl-phenyl-methyl, 3,4-dihydroxy-phenyl-ethyl, (3,4-dihydroxy-phenyl)-2-hydroxyethyl, (5-hydroxy-1H-indol-3-yl)ethylcyclic compounds having one or more cycles, consisting of a number of carbon atoms p comprised between 1 and 10, optionally substituted, R₁ and R₂, the same or different, are H, CH₃, alkyl, alkenyl, aryl or R1 are R₂ in a cyclic compound having one or more cycles, consisting of a number of carbon atoms m comprised between 1 and 10, optionally substituted, their stereoisomers, enantiomers, conformers and racemic mixture of different stereoisomers, for use as antimicrobial.

12. Compounds of formula (I): 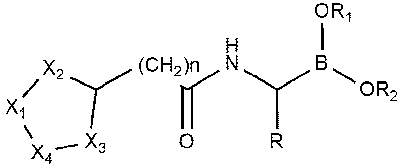 Wherein X₁ and X₂, the same or different, are CH, CH₂, the bond between X₁ and X₂ may be saturated or unsaturated, wherein X₃ and X₄ are the same and are S, n is an integer comprised between 1 and 10, R is H, alkyl selected from the group consisting of: methyl, ethyl, n-propyl, n-butyl, n-pentyl, n-hexyl, sec-butyl optionally substituted with a group selected from the group consisting of: adamantyl, i-propyl, sec-butyl, i-butyl, tert-butyl, i-pentyl, hydroxymethyl, 1-hydroxyethyl, sulfanylmethyl, 2-(methylsulfanyl)ethyl, carboxymethyl, carboxyethyl, 4-aminobutyl, 2-carbamoylethyl, 2-carbamoylmethyl, 2-methyl-3H-imidazole, 3-methyl-1H-indole, alkenyl selected from the group consisting of: ethenyl, 1-propenyl, 2-propenyl, 1-butenyl, 2-butenyl, 3-butenyl, isopentenyl, geranyl, farnesyl optionally substituted with a group selected from the group consisting of: ethenyl, 2-propenyl, isopentenyl, geranyl, farnesyl, aryl optionally substituted with a group selected from the group consisting of: benzyl, 4-hydroxybenzyl, 3-alkyl-1H-indole, 2-methyl-1H-imidazole, 3,4-dihydroxyphenyl-methyl, 4-(2-aminoethyl)benzene-1,2-diol, 4-hydroxy-3methyl-phenylmethyl, 4-hydroxy-3-isopropyl-phenylmethyl, 3,4-dihydroxy-phenyl-ethyl, (3,4-dihydroxy-phenyl)-2-hydroxyethyl, (5-hydroxy-1H-indol-3-yl)ethyl, cyclic compounds having one or more cycles, consisting of a number of carbon atoms p comprised between 1 and 10, optionally substituted, R₁ and R₂, the same or different, are H, CH₃, alkyl, alkenyl, aryl or R1 are R₂ in a cyclic compound having one or more cycles, consisting of a number of carbon atoms m comprised between 1 and 10, optionally substituted, their stereoisomers, enantiomers, conformers and racemic mixture of different stereoisomers, for use as antifungal.

13. Compounds of formula (I): 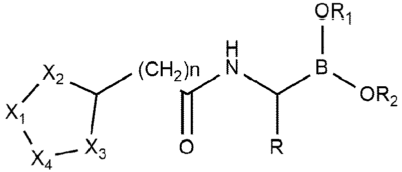 Wherein X₁ and X₂, the same or different, are CH, CH₂, the bond between X₁ and X₂ may be saturated or unsaturated, wherein X₃ and X₄ are the same and are s, n is an integer comprised between 1 and 10, R is H, alkyl selected from the group consisting of: methyl, ethyl, n-propyl, n-butyl, n-pentyl, n-hexyl, sec-butyl optionally substituted with a group selected from the group consisting of: adamantyl, i-propyl, sec-butyl, i-butyl, tert-butyl, i-pentyl, hydroxymethyl, 1-hydroxyethyl, sulfanylmethyl, 2-(methylsulfanyl)ethyl, carboxymethyl, carboxyethyl, 4-aminobutyl, 2-carbamoylethyl, 2-carbamoylmethyl, 2-methyl-3H-imidazole, 3-methyl-1H-indole, alkenyl selected from the group consisting of: ethenyl, 1-propenyl, 2-propenyl, 1-butenyl, 2-butenyl, 3-butenyl, isopentenyl, geranyl, farnesyl optionally substituted with a group selected from the group consisting of: ethenyl, 2-propenyl, isopentenyl, geranyl, farnesyl, aryl optionally substituted with a group selected from the group consisting of: benzyl, 4-hydroxybenzyl, 3-alkyl-1H-indole, 2-methyl-1H-imidazole, 3,4-dihydroxyphenyl-methyl, 4-(2-aminoethyl)benzene-1,2-diol, 4-hydroxy-3methyl-phenylmethyl, 4-hydroxy-3-isopropyl-phenylmethyl, 3,4-dihydroxy-phenyl-ethyl, (3,4-dihydroxy-phenyl)-2-hydroxyethyl, (5-hydroxy-1H-indol-3-yl)ethyl, cyclic compounds having one or more cycles, consisting of a number of carbon atoms p comprised between 1 and 10, optionally substituted, R₁ and R₂, the same or different, are H, CH₃, alkyl, alkenyl, aryl or R1 are R₂ in a cyclic compound having one or more cycles, consisting of a number of carbon atoms m comprised between 1 and 10, optionally substituted, their stereoisomers, enantiomers, conformers and racemic mixture of different stereoisomers, for use in the treatment of oxidative stress.

14. Compounds of formula (I): 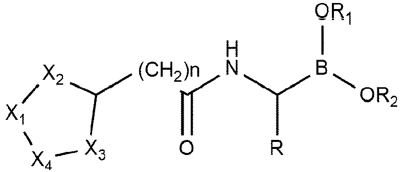 Wherein X₁ and X₂, the same or different, are CH, CH₂, the bond between X₁ and X₂ may be saturated or unsaturated, wherein X₃ and X₄ are the same and are S, n is an integer comprised between 1 and 10, R is H, alkyl selected from the group consisting of: methyl, ethyl, n-propyl, n-butyl, n-pentyl, n-hexyl, sec-butyl optionally substituted with a group selected from the group consisting of: adamantyl, i-propyl, sec-butyl, i-butyl, tert-butyl, i-pentyl, hydroxymethyl, 1-hydroxyethyl, sulfanylmethyl, 2-(methylsulfanyl)ethyl, carboxymethyl, carboxyethyl, 4-aminobutyl, 2-carbamoylethyl, 2-carbamoylmethyl, 2-methyl-3H-imidazole, 3-methyl-1H-indole, alkenyl selected from the group consisting of: ethenyl, 1-propenyl, 2-propenyl, 1-butenyl, 2-butenyl, 3-butenyl, isopentenyl, geranyl, farnesyl optionally substituted with a group selected from the group consisting of: ethenyl, 2-propenyl, isopentenyl, geranyl, farnesyl, aryl optionally substituted with a group selected from the group consisting of: benzyl, 4-hydroxybenzyl, 3-alkyl-1H-indole, 2-methyl-1H-imidazole, 3,4-dihydroxyphenyl-methyl, 4-(2-aminoethyl)benzene-1,2-diol, 4-hydroxy-3methyl-phenylmethyl, 4-hydroxy-3-isopropyl-phenylmethyl, 3,4-dihydroxy-phenyl-ethyl, (3,4-dihydroxy-phenyl)-2-hydroxyethyl, (5-hydroxy-1H-indol-3-yl)ethyl, cyclic compounds having one or more cycles, consisting of a number of carbon atoms p comprised between 1 and 10, optionally substituted, R₁ and R₂, the same or different, are H, CH₃, alkyl, alkenyl, aryl or R1 are R₂ in a cyclic compound having one or more cycles, consisting of a number of carbon atoms m comprised between 1 and 10, optionally substituted, their stereoisomers, enantiomers, conformers and racemic mixture of different stereoisomers, for use in the treatment of neurodegenerative diseases related to oxidative stress.

15. Compounds of formula (I): 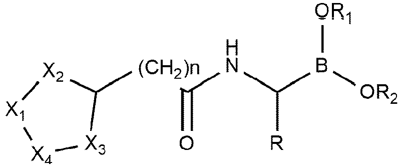 Wherein X₁ and X₂, the same or different, are CH, CH_{2,} the bond between X₁ and X₂ may be saturated or unsaturated, wherein X₃ e X₄ are the same and are s, n is an integer comprised between 1 and 10, R is H, alkyl selected from the group consisting of: methyl, ethyl, n-propyl, n-butyl, n-pentyl, n-hexyl, sec-butyl optionally substituted with a group selected from the group consisting of: adamantyl, i-propyl, sec-butyl, i-butyl, tert-butyl, i-pentyl, hydroxymethyl, 1-hydroxyethyl, sulfanylmethyl, 2-(methylsulfanyl)ethyl, carboxymethyl, carboxyethyl, 4-aminobutyl, 2-carbamoylethyl, 2-carbamoylmethyl, 2-methyl-3H-imidazole, 3-methyl-1H-indole, alkenyl selected from the group consisting of: ethenyl, 1-propenyl, 2-propenyl, 1-butenyl, 2-butenyl, 3-butenyl, isopentenyl, geranyl, farnesyl optionally substituted with a group selected from the group consisting of: ethenyl, 2-propenyl, isopentenyl, geranyl, farnesyl, aryl optionally substituted with a group selected from the group consisting of: benzyl, 4-hydroxybenzyl, 3-alkyl-1H-indole, 2-methyl-1H-imidazole, 3,4-dihydroxyphenyl-methyl, 4-(2-aminoethyl)benzene-1,2-diol, 4-hydroxy-3methyl-phenylmethyl, 4-hydroxy-3-isopropyl-phenylmethyl, 3,4-dihydroxy-phenyl-ethyl, (3,4-dihydroxy-phenyl)-2-hydroxyethyl, (5-hydroxy-1H-indol-3-yl)ethyl, cyclic compounds having one or more cycles, consisting of a number of carbon atoms p comprised between 1 and 10, optionally substituted, R₁ and R₂, the same or different, are H, CH₃, alkyl, alkenyl, aryl or R1 are R₂ in a cyclic compound having one or more cycles, consisting of a number of carbon atoms m comprised between 1 and 10, optionally substituted, their stereoisomers, enantiomers, conformers and racemic mixture of different stereoisomers, for use in the treatment of cardiovascular diseases related to oxidative stress.

16. Pharmaceutical composition comprising a pharmaceutical active amount of compounds of formula (I) 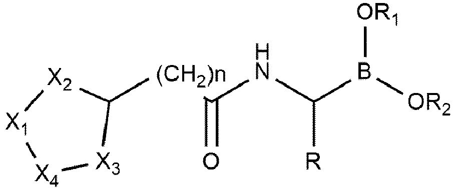 Wherein X₁ and X₂, the same or different, are CH, CH₂, the bond between X₁ and X₂ may be saturated or unsaturated, wherein X₃ and X₄ are the same and are S, n is an integer comprised between 1 and 10, R is H, alkyl selected from the group consisting of: methyl, ethyl, n-propyl, n-butyl, n-pentyl, n-hexyl, sec-butyl optionally substituted with a group selected from the group consisting of: adamantyl, i-propyl, sec-butyl, i-butyl, tert-butyl, i-pentyl, hydroxymethyl, 1-hydroxyethyl, sulfanylmethyl, 2-(methylsulfanyl)ethyl, carboxymethyl, carboxyethyl, 4-aminobutyl, 2-carbamoylethyl, 2-carbamoylmethyl, 2-methyl-3H-imidazole, 3-methyl-1H-indole, alkenyl selected from the group consisting of: ethenyl, 1-propenyl, 2-propenyl, 1-butenyl, 2-butenyl, 3-butenyl, isopentenyl, geranyl, farnesyl optionally substituted with a group selected from the group consisting of: ethenyl, 2-propenyl, isopentenyl, geranyl, farnesyl, aryl optionally substituted with a group selected from the group consisting of: benzyl, 4-hydroxybenzyl, 3-alkyl-1H-indole, 2-methyl-1H-imidazole, 3,4-dihydroxyphenyl-methyl, 4-(2-aminoethyl)benzene-1,2-diol, 4-hydroxy-3methyl-phenylmethyl, 4-hydroxy-3-isopropyl-phenylmethyl, 3,4-dihydroxy-phenyl-ethyl, (3,4-dihydroxy-phenyl)-2-hydroxyethyl, (5-hydroxy-1H-indol-3-yl)ethyl, cyclic compounds having one or more cycles, consisting of a number of carbon atoms p comprised between 1 and 10, optionally substituted, R₁ and R₂, the same or different, are H, CH₃, alkyl, alkenyl, aryl or R1 are R₂ in a cyclic compound having one or more cycles, consisting of a number of carbon atoms m comprised between 1 and 10, optionally substituted, their stereoisomers, enantiomers, conformers and racemic mixture of different stereoisomers and suitable pharmaceutical acceptable excipients.

## Patentansprüche

1. Verbindungen der Formel (I): 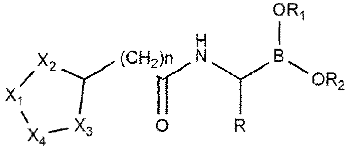 worin
X₁ und X₂, die gleich oder verschieden sind, CH, CH₂ sind,
die Bindung zwischen X₁ und X₂ gesättigt oder ungesättigt sein kann,
X₃ und X₄ gleich sind und S sind,
n eine ganze Zahl ist, die zwischen 1 und 10 umfasst,
R H, Alkyl ist, ausgewählt aus der Gruppe, bestehend aus: Methyl, Ethyl, n-Propyl, n-Butyl, n-Pentyl, n-Hexyl, sec-Butyl, optional substituiert mit einer Gruppe, ausgewählt aus der Gruppe, bestehend aus: Adamantyl, i-Propyl, sec-Butyl, i-Butyl, tert-Butyl, i-Pentyl, Hydroxymethyl, 1-Hydroxyethyl, Sulfanylmethyl, 2-(Methylsulfanyl)ethyl, Carboxymethyl, Carboxyethyl, 4-Aminobutyl, 2-Carbamoylethyl, 2-Carbamoylmethyl, 2-Methyl-3H-imidazol, 3-Methyl-1H-indol, Alkenyl, ausgewählt aus der Gruppe, bestehend aus: Ethenyl, 1-Propenyl, 2-Propenyl, 1-Butenyl, 2-Butenyl, 3-Butenyl, Isopentenyl, Geranyl, Farnesyl, optional substituiert mit einer Gruppe, ausgewählt aus der Gruppe, bestehend aus: Ethenyl, 2-Propenyl, Isopentenyl, Geranyl, Farnesyl, Aryl, optional substituiert mit einer Gruppe, ausgewählt aus der Gruppe, bestehend aus: Benzyl, 4-Hydroxybenzyl, 3-Alkyl-1H-indol, 2-Methyl-1H-imidazol, 3,4-Dihydroxyphenylmethyl, 4-(2-Aminoethyl)benzen-1,2-diol, 4-Hydroxy-3-methylphenylmethyl, 4-Hydroxy-3-isopropylphenylmethyl, 3,4-Dihydroxyphenylethyl, (3,4-Dihydroxyphenyl)-2-hydroxyethyl, (5-Hydroxy-1H-indol-3-yl)ethyl, zyklischen Verbindungen, die einen oder mehrere Ringe haben, bestehend aus einer Anzahl von Kohlenstoffatomen p, die zwischen 1 und 10 umfasst, optional substituiert,
R₁ und R₂, die gleich oder verschieden sind, H, CH₃, Alkyl, Alkenyl, Aryl sind, oder R₁ in einer zyklischen Verbindung R₂ sind, die einen Ring oder mehrere Ringe hat, bestehend aus einer Anzahl von Kohlenstoffatomen m, die zwischen 1 und 10 umfasst, optional substituiert,
ihre Stereoisomere, Enantiomere, Konformere und racemische Mischung verschiedener Stereoisomere.

2. Verbindungen der Formel (I), gemäß Anspruch 1, worin X₁-X₂ CH₂-CH₂, CH=CH ist.

3. Verbindungen der Formel (I), gemäß Anspruch 1, worin n 4 ist.

4. Verbindungen der Formel (I), gemäß Anspruch 1, worin R H ist.

5. Verbindungen der Formel (I), gemäß Anspruch 1, ausgewählt aus der Gruppe, bestehend aus:
5-((S)-1,2-Dithiolan-3-yl)-*N*-(((3aR)-3a,5,5-trimethylhexahydro-4,6-methanbenzo[d][1,3,2,]dioxaborol-2-yl)methyl)pentanamid,
(*S*)-((5-(1,2-Dithiolan-3-yl)pentanamid)methyl)boronsäure,
((*S*)-1-(5-((R)-1,2-Dithiolan-3-yl)pentanamid)ethyl)boronsäure,
((S)-1-(5-((R)-1,2-Dithiolan-3-yl)pentanamid)-3-methylbutyl)boronsäure,
((S)-1-(5-((R)-1,2-Dithiolan-3-yl)pentanamid)-2-mercaptoethyl)-boronsäure,
((1S)-1-(5-(1,2-Dithiolan-3-yl)pentanamid)-2-(3,4-dihydroxyphenyl)ethyl)boronsäure,
((S)-1-(5-((R)-1,2-Dithiolan-3-yl)pentanamid)-2-(4-hydroxy-3-methylphenyl)ethyl)boronsäure,
((S)-1-(5-((R)-1,2-Dithiolan-3-yl)pentanamid)-2-(4-hydroxy-3-isopropylphenyl)ethyl)boronsäure,
((S)-1-(5-((R)-1,2-Dithiolan-3-yl)pentanamid)-3-(3,4-dihydroxyphenyl)propyl)boronsäure,
((3S)-1-(5-((R)-1,2-Dithiolan-3-yl)pentanamid)-3-(3,4-dihydroxyphenyl)-3-hydroxypropyl)boronsäure,
((S)-1-(5-((R)-1,2-Dithiolan-3-yl)pentanamid)-2-hydroxyethyl)-boronsäure,
(1-(5-((R)-1,2-dithiolan-3-yl)pentanamid)-4-methylpent-3-en-1 - yl)boronsäure,
((S)-1-(5-((R)-1,2-Dithiolan-3-yl)pentanamid)-3-(5-hydroxy-1H-indol-3-yl)propyl)boronsäure,
5-((R)-1,2-Dithiolan-3-yl)-N-((1S)-1-(3a,5,5-trimethylhexahydro-1H-4,6-methanbenzo[c]borol-2(3H)-yl)ethyl)pentanamid,
5-((R)-1,2-Dithiolan-3-yl)-N-((1S)-3-methyl-1-(3a,5,5-trimethylhexahydro-4,6-methanbenzo[d][1,3,2]dioxaborol-2-yl)butyl)pentanamid,
N-((1S)-2-(3,4-dihydroxyphenyl)-1-(3a,5,5-trimethylhexahydro-4,6-methanbenzo[d][1,3,2]dioxaborol-2-yl)ethyl)-5-(((R)-1,2-dithiolan-3-yl)pentanamid,
5-((R)-1,2-Dithiolan-3-yl)-N-((1S)-2-mercapto-1-(3a,5,5-trimethylhexahydro-4,6-methanbenzo[d][1,3,2]dioxaborol-2-yl)ethyl)pentanamid,
5-((R)-1,2-Dithiolan-3-yl)-N-((1S)-2-(4-hydroxy-3-isopropylphenyl)-1-(3a,5,5-trimethylhexahydro-4,6-methanbenzo[d][1,3,2]dioxaborol-2-yl)ethyl)pentanamid,
5-((R)-1,2-Dithiolan-3-yl)-N-((1S)-2-(4-hydroxy-3-methylphenyl)-1-(3a,5,5-trimethylhexahydro-4,6-methanbenzo[d][1,3,2]dioxaborol-2-yl)ethyl)pentanamid,
N-((3S)-3-(3,4-Dihydroxyphenyl)-3-hydroxy-1-(3a,5,5-trimethylhexahydro-4,6-methanbenzo[d][1,3,2]dioxaborol-2-yl)propyl)-5-((R)-1,2-dithiolan-3-yl)pentanamid,
N-((1S)-3-(3,4-Dihydroxyphenyl)-1-(3a,5,5-trimethylhexahydro-4,6-methanbenzo[d][1,3,2]dioxaborol-2-yl)propyl)-5-((R)-1,2-dithiolan-3-yl)pentanamid,
5-((R)-1,2-Dithiolan-3-yl)-N-((1S)-2-hydroxy-1-(3a,5,5-trimethylhexahydro-4,6-methanbenzo[d][1,3,2]dioxaborol-2-yl)ethyl)pentanamid,
5-((R)-1,2-Dithiolan-3-yl)-N-((1S)-3-(5-hydroxy-1H-indol-3-yl)-1-(3a,5,5-trimethylhexahydro-4,6-methanobenzo[d][1,3,2]dioxaborol-2-yl)propyl)pentanamid,
5-((R)-1,2-Dithiolan-3-yl)-N-(4-methyl-1-(3a,5,5-trimethylhexahydro-4,6-methanbenzo[d][1,3,2]dioxaborol-2-yl)pent-3-en-1-yl)pentanamid,

6. Verbindungen der Formel (I): 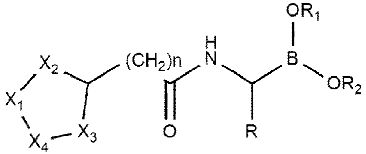 worin X₁ und X₂, die gleich oder verschieden sind, CH, CH₂ sind, die Bindung zwischen X₁ und X₂ gesättigt oder ungesättigt sein kann, worin X₃ und X₄ gleich sind und S sind, n eine ganze Zahl ist, die zwischen 1 und 10 umfasst, R H, Alkyl ist, ausgewählt aus der Gruppe, bestehend aus: Methyl, Ethyl, n-Propyl, n-Butyl, n-Pentyl, n-Hexyl, sec-Butyl, optional substituiert mit einer Gruppe, ausgewählt aus der Gruppe, bestehend aus: Adamantyl, i-Propyl, sec-Butyl, i-Butyl, tert-Butyl, i-Pentyl, Hydroxymethyl, 1-Hydroxyethyl, Sulfanylmethyl, 2-(Methylsulfanyl)ethyl, Carboxymethyl, Carboxyethyl, 4-Aminobutyl, 2-Carbamoylethyl, 2-Carbamoylmethyl, 2-Methyl-3H-imidazol, 3-Methyl-1H-indol, Alkenyl, ausgewählt aus der Gruppe, bestehend aus: Ethenyl, 1-Propenyl, 2-Propenyl, 1-Butenyl, 2-Butenyl, 3-Butenyl, Isopentenyl, Geranyl, Farnesyl, optional substituiert mit einer Gruppe, ausgewählt aus der Gruppe, bestehend aus: Ethenyl, 2-Propenyl, Isopentenyl, Geranyl, Farnesyl, Aryl, optional substituiert mit einer Gruppe, ausgewählt aus der Gruppe, bestehend aus: Benzyl, 4-Hydroxybenzyl, 3-Alkyl-1H-indol, 2-Methyl-1H-imidazol, 3,4-Dihydroxyphenylmethyl, 4-(2-Aminoethyl)benzen-1,2-diol, 4-Hydroxy-3-methylphenylmethyl, 4-Hydroxy-3-isopropylphenylmethyl, 3,4-Dihydroxyphenylethyl, (3,4-Dihydroxyphenyl)-2-hydroxyethyl, (5-Hydroxy-1H-indol-3-yl)ethyl, zyklischen Verbindungen, die einen oder mehrere Ringe haben, bestehend aus einer Anzahl von Kohlenstoffatomen p, die zwischen 1 und 10 umfasst, optional substituiert, R₁ und R₂, die gleich oder verschieden sind, H, CH₃, Alkyl, Alkenyl, Aryl sind, oder R₁ sind R₂ in einer zyklischen Verbindung, die einen Ring oder mehrere Ringe hat, bestehend aus einer Anzahl von Kohlenstoffatomen m, die zwischen 1 und 10 umfasst, optional substituiert, ihre Stereoisomere, Enantiomere, Konformere und racemische Mischung von verschiedenen Stereoisomeren, zur Verwendung als Medikament.

7. Verbindungen der Formel (I) 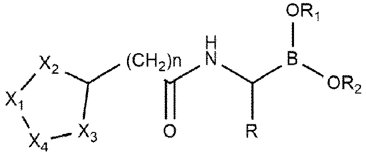 worin X₁ und X₂, die gleich oder verschieden sind, CH, CH₂ sind, die Bindung zwischen X₁ und X₂ gesättigt oder ungesättigt sein kann, worin X₃ und X₄ gleich sind und S sind, n eine ganze Zahl ist, die zwischen 1 und 10 umfasst, R H ist, Alkyl, ausgewählt aus der Gruppe, bestehend aus: Methyl, Ethyl, n-Propyl, n-Butyl, n-Pentyl, n-Hexyl, sec-Butyl, optional substituiert mit einer Gruppe, ausgewählt aus der Gruppe, bestehend aus: Adamantyl, i-Propyl, sec-Butyl, i-Butyl, tert-Butyl, i-Pentyl, Hydroxymethyl, 1-Hydroxyethyl, Sulfanylmethyl, 2-(Methylsulfanyl)ethyl, Carboxymethyl, Carboxyethyl, 4-Aminobutyl, 2-Carbamoylethyl, 2-Carbamoylmethyl, 2-Methyl-3H-imidazol, 3-Methyl-1H-Indol, Alkenyl, ausgewählt aus der Gruppe, bestehend aus: Ethenyl, 1-Propenyl, 2-Propenyl, 1-Butenyl, 2-Butenyl, 3-Butenyl, Isopentenyl, Geranyl, Farnesyl, optional substituiert mit einer Gruppe, ausgewählt aus der Gruppe, bestehend aus: Ethenyl, 2-Propenyl, Isopentenyl, Geranyl, Farnesyl, Aryl, optional substituiert mit einer Gruppe, ausgewählt aus der Gruppe, bestehend aus: Benzyl, 4-Hydroxybenzyl, 3-Alkyl-1H-indol, 2-Methyl-1H-imidazol, 3,4-Dihydroxyphenylmethyl, 4-(2-Aminoethyl)benzen-1,2-diol, 4-Hydroxy-3-methylphenylmethyl, 4-Hydroxy-3-isopropylphenylmethyl, 3,4-Dihydroxyphenylethyl, (3,4-Dihydroxyphenyl)-2-hydroxyethyl, (5-Hydroxy-1H-indol-3-yl)ethyl, zyklischen Verbindungen, die einen Ring oder mehrere Ringe haben, bestehend aus einer Anzahl von Kohlenstoffatomen p, die zwischen 1 und 10 umfasst, optional substituiert, R₁ und R₂, die gleich oder verschieden sind, H, CH₃, Alkyl, Alkenyl, Aryl sind, oder R₁ sind R₂ in einer zyklischen Verbindung, die einen oder mehrere Ringe hat, bestehend aus einer Anzahl von Kohlenstoffatomen m, die zwischen 1 und 10 umfasst, optional substituiert, ihre Stereoisomere, Enantiomere, Konformere und racemische Mischung von verschiedenen Stereoisomeren, zur Verwendung als Neuroprotektor.

8. Verbindungen der Formel (I) 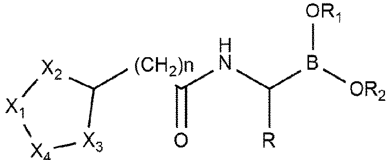 worin X₁ und X₂, die gleich oder verschieden sind, CH, CH₂ sind, die Bindung zwischen X₁ und X₂ gesättigt oder ungesättigt sein kann, worin X₃ und X₄ gleich sind und S sind, n eine ganze Zahl ist, die zwischen 1 und 10 umfasst, R H, Alkyl ist, ausgewählt aus der Gruppe, bestehend aus: Methyl, Ethyl, n-Propyl, n-Butyl, n-Pentyl, n-Hexyl, sec-Butyl, optional substituiert mit einer Gruppe, ausgewählt aus der Gruppe, bestehend aus: Adamantyl, i-Propyl, sec-Butyl, i-Butyl, tert-Butyl, i-Pentyl, Hydroxymethyl, 1-Hydroxyethyl, Sulfanylmethyl, 2-(Methylsulfanyl)ethyl, Carboxymethyl, Carboxyethyl, 4-Aminobutyl, 2-Carbamoylethyl, 2-Carbamoylmethyl, 2-Methyl-3H-imidazol, 3-Methyl-1H-Indol, Alkenyl, ausgewählt aus der Gruppe, bestehend aus: Ethenyl, 1-Propenyl, 2-Propenyl, 1-Butenyl, 2-Butenyl, 3-Butenyl, Isopentenyl, Geranyl, Farnesyl, optional substituiert mit einer Gruppe, ausgewählt aus der Gruppe, bestehend aus: Ethenyl, 2-Propenyl, Isopentenyl, Geranyl, Farnesyl, Aryl, optional substituiert mit einer Gruppe, ausgewählt aus der Gruppe, bestehend aus: Benzyl, 4-Hydroxybenzyl, 3-Alkyl-1H-indol, 2-Methyl-1H-imidazol, 3,4-Dihydroxyphenylmethyl, 4-(2-Aminoethyl)benzen-1,2-diol, 4-Hydroxy-3-methylphenylmethyl, 4-Hydroxy-3-isopropylphenylmethyl, 3,4-Dihydroxyphenylethyl, (3,4-Dihydroxyphenyl)-2-hydroxyethyl, (5-Hydroxy-1H-indol-3-yl)ethyl, zyklischen Verbindungen, die einen Ring oder mehrere Ringe haben, bestehend aus einer Anzahl von Kohlenstoffatomen p, die zwischen 1 und 10 umfasst, optional substituiert, R₁ und R₂, die gleich oder verschieden sind, H, CH₃, Alkyl, Alkenyl, Aryl sind oder R₁ sind R₂ in einer zyklischen Verbindung, die einen oder mehrere Ringe hat, bestehend aus einer Anzahl von Kohlenstoffatomen m, die zwischen 1 und 10 umfasst, optional substituiert, ihre Stereoisomere, Enantiomere, Konformere und racemische Mischung von verschiedenen Stereoisomeren, zur Verwendung als Blutplättchenaggregationshemmer.

9. Verbindungen der Formel (I) 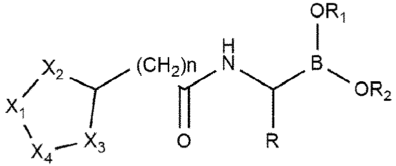 worin X₁ und X₂, die gleich oder verschieden sind, CH, CH₂ sind, die Bindung zwischen X₁ und X₂ gesättigt oder ungesättigt sein kann, worin X₃ und X₄ gleich sind und S sind, n eine ganze Zahl ist, die zwischen 1 und 10 umfasst, R H, Alkyl ist, ausgewählt aus der Gruppe, bestehend aus: Methyl, Ethyl, n-Propyl, n-Butyl, n-Pentyl, n-Hexyl, sec-Butyl, optional substituiert mit einer Gruppe, ausgewählt aus der Gruppe, bestehend aus: Adamantyl, i-Propyl, sec-Butyl, i-Butyl, tert-Butyl, i-Pentyl, Hydroxymethyl, 1-Hydroxyethyl, Sulfanylmethyl, 2-(Methylsulfanyl)ethyl, Carboxymethyl, Carboxyethyl, 4-Aminobutyl, 2-Carbamoylethyl, 2-Carbamoylmethyl, 2-Methyl-3H-imidazol, 3-Methyl-1H-Indol, Alkenyl, ausgewählt aus der Gruppe, bestehend aus: Ethenyl, 1-Propenyl, 2-Propenyl, 1-Butenyl, 2-Butenyl, 3-Butenyl, Isopentenyl, Geranyl, Farnesyl, optional substituiert mit einer Gruppe, ausgewählt aus der Gruppe, bestehend aus: Ethenyl, 2-Propenyl, Isopentenyl, Geranyl, Farnesyl, Aryl, optional substituiert mit einer Gruppe, ausgewählt aus der Gruppe, bestehend aus: Benzyl, 4-Hydroxybenzyl, 3-Alkyl-1H-indol, 2-Methyl-1H-imidazol, 3,4-Dihydroxyphenylmethyl, 4-(2-Aminoethyl)benzen-1,2-diol, 4-Hydroxy-3-methylphenylmethyl, 4-Hydroxy-3-isopropylphenylmethyl, 3,4-Dihydroxyphenylethyl, (3,4-Dihydroxyphenyl)-2-hydroxyethyl, (5-Hydroxy-1H-indol-3-yl)ethyl, zyklischen Verbindungen, die einen oder mehrere Ringe haben, bestehend aus einer Anzahl von Kohlenstoffatomen p, die zwischen 1 und 10 umfasst, optional substituiert, R₁ und R₂, die gleich oder verschieden sind, H, CH₃, Alkyl, Alkenyl, Aryl sind, oder R₁ sind R₂ in einer zyklischen Verbindung, die einen oder mehrere Ringe hat, bestehend aus einer Anzahl von Kohlenstoffatomen m, die zwischen 1 und 10 umfasst, optional substituiert, ihre Stereoisomere, Enantiomere, Konformere und racemische Mischung von verschiedenen Stereoisomeren, zur Verwendung als chemopräventives Agens.

10. Verbindungen der Formel (I) 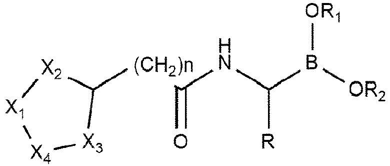 worin X₁ und X₂, die gleich oder verschieden sind, CH, CH₂ sind, die Bindung zwischen X₁ und X₂ gesättigt oder ungesättigt sein kann, worin X₃ und X₄ gleich sind und S sind, n eine ganze Zahl ist, die zwischen 1 und 10 umfasst, R H, Alkyl ist, ausgewählt aus der Gruppe, bestehend aus: Methyl, Ethyl, n-Propyl, n-Butyl, n-Pentyl, n-Hexyl, sec-Butyl, optional substituiert mit einer Gruppe, ausgewählt aus der Gruppe, bestehend aus: Adamantyl, i-Propyl, sec-Butyl, i-Butyl, tert-Butyl, i-Pentyl, Hydroxymethyl, 1-Hydroxyethyl, Sulfanylmethyl, 2-(Methylsulfanyl)ethyl, Carboxymethyl, Carboxyethyl, 4-Aminobutyl, 2-Carbamoylethyl, 2-Carbamoylmethyl, 2-Methyl-3H-imidazol, 3-Methyl-1H-Indol, Alkenyl, ausgewählt aus der Gruppe, bestehend aus: Ethenyl, 1-Propenyl, 2-Propenyl, 1-Butenyl, 2-Butenyl, 3-Butenyl, Isopentenyl, Geranyl, Farnesyl, optional substituiert mit einer Gruppe, ausgewählt aus der Gruppe, bestehend aus: Ethenyl, 2-Propenyl, Isopentenyl, Geranyl, Farnesyl, Aryl, optional substituiert mit einer Gruppe, ausgewählt aus der Gruppe, bestehend aus: Benzyl, 4-Hydroxybenzyl, 3-Alkyl-1H-indol, 2-Methyl-1H-imidazol, 3,4-Dihydroxyphenylmethyl, 4-(2-Aminoethyl)benzen-1,2-diol, 4-Hydroxy-3-methylphenylmethyl, 4-Hydroxy-3-isopropylphenylmethyl, 3,4-Dihydroxyphenylethyl, (3,4-Dihydroxyphenyl)-2-hydroxyethyl, (5-Hydroxy-1H-indol-3-yl)ethyl, zyklischen Verbindungen, die einen oder mehrere Ringe haben, bestehend aus einer Anzahl von Kohlenstoffatomen p, die zwischen 1 und 10 umfasst, optional substituiert, R₁ und R₂, die gleich oder verschieden sind, H, CH₃, Alkyl, Alkenyl, Aryl sind, oder R₁ sind R₂ in einer zyklischen Verbindung, die einen oder mehrere Ringe hat, bestehend aus einer Anzahl von Kohlenstoffatomen m, die zwischen 1 und 10 umfasst, optional substituiert, ihre Stereoisomere, Enantiomere, Konformere und racemische Mischung von verschiedenen Stereoisomeren, zur Verwendung als Antitumor-Agens.

11. Verbindungen der Formel (I) 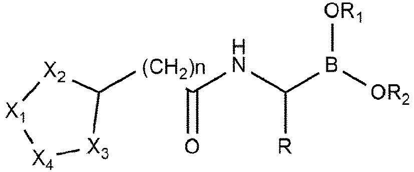 worin X₁ und X₂, die gleich oder verschieden sind, CH, CH₂ sind, die Bindung zwischen X₁ und X₂ gesättigt oder ungesättigt sein kann, worin X₃ und X₄ gleich sind und S sind, n eine ganze Zahl ist, die zwischen 1 und 10 umfasst, R H, Alkyl ist, ausgewählt aus der Gruppe, bestehend aus: Methyl, Ethyl, n-Propyl, n-Butyl, n-Pentyl, n-Hexyl, sec-Butyl, optional substituiert mit einer Gruppe, ausgewählt aus der Gruppe, bestehend aus: Adamantyl, i-Propyl, sec-Butyl, i-Butyl, tert-Butyl, i-Pentyl, Hydroxymethyl, 1-Hydroxyethyl, Sulfanylmethyl, 2-(Methylsulfanyl)ethyl, Carboxymethyl, Carboxyethyl, 4-Aminobutyl, 2-Carbamoylethyl, 2-Carbamoylmethyl, 2-Methyl-3H-imidazol, 3-Methyl-1H-Indol, Alkenyl, ausgewählt aus der Gruppe, bestehend aus: Ethenyl, 1-Propenyl, 2-Propenyl, 1-Butenyl, 2-Butenyl, 3-Butenyl, Isopentenyl, Geranyl, Farnesyl, optional substituiert mit einer Gruppe, ausgewählt aus der Gruppe, bestehend aus: Ethenyl, 2-Propenyl, Isopentenyl, Geranyl, Farnesyl, Aryl, optional substituiert mit einer Gruppe, ausgewählt aus der Gruppe, bestehend aus: Benzyl, 4-Hydroxybenzyl, 3-Alkyl-1H-indol, 2-Methyl-1H-imidazol, 3,4-Dihydroxyphenylmethyl, 4-(2-Aminoethyl)benzen-1,2-diol, 4-Hydroxy-3-methylphenylmethyl, 4-Hydroxy-3-isopropylphenylmethyl, 3,4-Dihydroxyphenylethyl, (3,4-Dihydroxyphenyl)-2-hydroxyethyl, (5-Hydroxy-1H-indol-3-yl)ethyl, zyklischen Verbindungen, die einen oder mehrere Ringe haben, bestehend aus einer Anzahl von Kohlenstoffatomen p, die zwischen 1 und 10 umfasst, optional substituiert, R₁ und R₂, die gleich oder verschieden sind, H, CH₃, Alkyl, Alkenyl, Aryl sind, oder R₁ sind R₂ in einer zyklischen Verbindung, die einen oder mehrere Ringe hat, bestehend aus einer Anzahl von Kohlenstoffatomen m, die zwischen 1 und 10 umfasst, optional substituiert, ihre Stereoisomere, Enantiomere, Konformere und racemische Mischung von verschiedenen Stereoisomeren, zur Verwendung als antimikrobielles Agens.

12. Verbindungen der Formel (I): 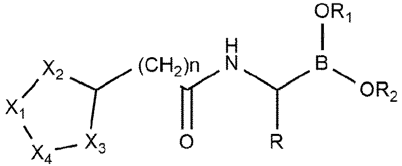 worin X₁ und X₂, die gleich oder verschieden sind, CH, CH₂ sind, die Bindung zwischen X₁ und X₂ gesättigt oder ungesättigt sein kann, worin X₃ und X₄ gleich sind und S sind, n eine ganze Zahl ist, die zwischen 1 und 10 umfasst, R H, Alkyl ist, ausgewählt aus der Gruppe, bestehend aus: Methyl, Ethyl, n-Propyl, n-Butyl, n-Pentyl, n-Hexyl, sec-Butyl, optional substituiert mit einer Gruppe, ausgewählt aus der Gruppe, bestehend aus: Adamantyl, i-Propyl, sec-Butyl, i-Butyl, tert-Butyl, i-Pentyl, Hydroxymethyl, 1-Hydroxyethyl, Sulfanylmethyl, 2-(Methylsulfanyl)ethyl, Carboxymethyl, Carboxyethyl, 4-Aminobutyl, 2-Carbamoylethyl, 2-Carbamoylmethyl, 2-Methyl-3H-imidazol, 3-Methyl-1H-Indol, Alkenyl, ausgewählt aus der Gruppe, bestehend aus: Ethenyl, 1-Propenyl, 2-Propenyl, 1-Butenyl, 2-Butenyl, 3-Butenyl, Isopentenyl, Geranyl, Farnesyl, optional substituiert mit einer Gruppe, ausgewählt aus der Gruppe, bestehend aus: Ethenyl, 2-Propenyl, Isopentenyl, Geranyl, Farnesyl, Aryl, optional substituiert mit einer Gruppe, ausgewählt aus der Gruppe, bestehend aus: Benzyl, 4-Hydroxybenzyl, 3-Alkyl-1H-indol, 2-Methyl-1H-imidazol, 3,4-Dihydroxyphenylmethyl, 4-(2-Aminoethyl)benzen-1,2-diol, 4-Hydroxy-3-methylphenylmethyl, 4-Hydroxy-3-isopropylphenylmethyl, 3,4-Dihydroxyphenylethyl, (3,4-Dihydroxyphenyl)-2-hydroxyethyl, (5-Hydroxy-1H-indol-3-yl)ethyl, zyklischen Verbindungen, die einen oder mehrere Ringe haben, bestehend aus einer Anzahl von Kohlenstoffatomen p, die zwischen 1 und 10 umfasst, optional substituiert, R₁ und R₂, die gleich oder verschieden sind, H, CH₃, Alkyl, Alkenyl, Aryl sind, oder R₁ sind R₂ in einer zyklischen Verbindung, die einen oder mehrere Ringe hat, bestehend aus einer Anzahl von Kohlenstoffatomen m, die zwischen 1 und 10 umfasst, optional substituiert, ihre Stereoisomere, Enantiomere, Konformere und racemische Mischung von verschiedenen Stereoisomeren, zur Verwendung als antifungales Agens.

13. Verbindungen der Formel (I): 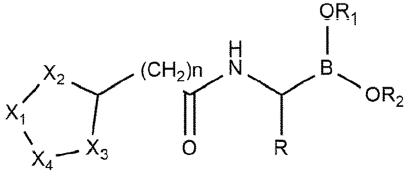 worin X₁ und X₂, die gleich oder verschieden sind, CH, CH₂ sind, die Bindung zwischen X₁ und X₂ gesättigt oder ungesättigt sein kann, worin X₃ und X₄ gleich sind und S sind, n eine ganze Zahl ist, die zwischen 1 und 10 umfasst, R H, Alkyl ist, ausgewählt aus der Gruppe, bestehend aus: Methyl, Ethyl, n-Propyl, n-Butyl, n-Pentyl, n-Hexyl, sec-Butyl, optional substituiert mit einer Gruppe, ausgewählt aus der Gruppe, bestehend aus: Adamantyl, i-Propyl, sec-Butyl, i-Butyl, tert-Butyl, i-Pentyl, Hydroxymethyl, 1-Hydroxyethyl, Sulfanylmethyl, 2-(Methylsulfanyl)ethyl, Carboxymethyl, Carboxyethyl, 4-Aminobutyl, 2-Carbamoylethyl, 2-Carbamoylmethyl, 2-Methyl-3H-imidazol, 3-Methyl-1H-Indol, Alkenyl, ausgewählt aus der Gruppe, bestehend aus: Ethenyl, 1-Propenyl, 2-Propenyl, 1-Butenyl, 2-Butenyl, 3-Butenyl, Isopentenyl, Geranyl, Farnesyl, optional substituiert mit einer Gruppe, ausgewählt aus der Gruppe, bestehend aus: Ethenyl, 2-Propenyl, Isopentenyl, Geranyl, Farnesyl, Aryl, optional substituiert mit einer Gruppe, ausgewählt aus der Gruppe, bestehend aus: Benzyl, 4-Hydroxybenzyl, 3-Alkyl-1H-indol, 2-Methyl-1H-imidazol, 3,4-Dihydroxyphenylmethyl, 4-(2-Aminoethyl)benzen-1,2-diol, 4-Hydroxy-3-methylphenylmethyl, 4-Hydroxy-3-isopropylphenylmethyl, 3,4-Dihydroxyphenylethyl, (3,4-Dihydroxyphenyl)-2-hydroxyethyl, (5-Hydroxy-1H-indol-3-yl)ethyl, zyklischen Verbindungen, die einen oder mehrere Ringe haben, bestehend aus einer Anzahl von Kohlenstoffatomen p, die zwischen 1 und 10 umfasst, optional substituiert, R₁ und R₂, die gleich oder verschieden sind, H, CH₃, Alkyl, Alkenyl, Aryl sind, oder R₁ sind R₂ in einer zyklischen Verbindung, die einen oder mehrere Ringe hat, bestehend aus einer Anzahl von Kohlenstoffatomen m, die zwischen 1 und 10 umfasst, optional substituiert, ihre Stereoisomere, Enantiomere, Konformere und racemische Mischung von verschiedenen Stereoisomeren, zur Verwendung bei der Behandlung von oxidativem Stress.

14. Verbindungen der Formel (I): 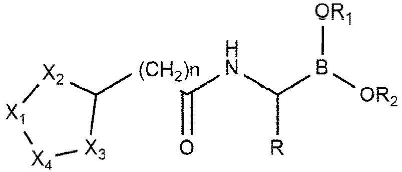 worin X₁ und X₂, die gleich oder verschieden sind, CH, CH₂ sind, die Bindung zwischen X₁ und X₂ gesättigt oder ungesättigt sein kann, worin X₃ und X₄ gleich sind und S sind, n eine ganze Zahl ist, die zwischen 1 und 10 umfasst, R H, Alkyl ist, ausgewählt aus der Gruppe, bestehend aus: Methyl, Ethyl, n-Propyl, n-Butyl, n-Pentyl, n-Hexyl, sec-Butyl, optional substituiert mit einer Gruppe, ausgewählt aus der Gruppe, bestehend aus: Adamantyl, i-Propyl, sec-Butyl, i-Butyl, tert-Butyl, i-Pentyl, Hydroxymethyl, 1-Hydroxyethyl, Sulfanylmethyl, 2-(Methylsulfanyl)ethyl, Carboxymethyl, Carboxyethyl, 4-Aminobutyl, 2-Carbamoylethyl, 2-Carbamoylmethyl, 2-Methyl-3H-imidazol, 3-Methyl-1H-Indol, Alkenyl, ausgewählt aus der Gruppe, bestehend aus: Ethenyl, 1-Propenyl, 2-Propenyl, 1-Butenyl, 2-Butenyl, 3-Butenyl, Isopentenyl, Geranyl, Farnesyl, optional substituiert mit einer Gruppe, ausgewählt aus der Gruppe, bestehend aus: Ethenyl, 2-Propenyl, Isopentenyl, Geranyl, Farnesyl, Aryl, optional substituiert mit einer Gruppe, ausgewählt aus der Gruppe, bestehend aus: Benzyl, 4-Hydroxybenzyl, 3-Alkyl-1H-indol, 2-Methyl-1H-imidazol, 3,4-Dihydroxyphenylmethyl, 4-(2-Aminoethyl)benzen-1,2-diol, 4-Hydroxy-3-methylphenylmethyl, 4-Hydroxy-3-isopropylphenylmethyl, 3,4-Dihydroxyphenylethyl, (3,4-Dihydroxyphenyl)-2-hydroxyethyl, (5-Hydroxy-1H-indol-3-yl)ethyl, zyklischen Verbindungen, die einen oder mehrere Ringe haben, bestehend aus einer Anzahl von Kohlenstoffatomen p, die zwischen 1 und 10 umfasst, optional substituiert, R₁ und R₂, die gleich oder verschieden sind, H, CH₃, Alkyl, Alkenyl, Aryl sind, oder R₁ sind R₂ in einer zyklischen Verbindung, die einen oder mehrere Ringe hat, bestehend aus einer Anzahl von Kohlenstoffatomen m, die zwischen 1 und 10 umfasst, optional substituiert, ihre Stereoisomere, Enantiomere, Konformere und racemische Mischung von verschiedenen Stereoisomeren, zur Verwendung bei der Behandlung von neurodegenerativen Krankheiten, die mit oxidativem Stress verbunden sind.

15. Verbindungen der Formel (I): 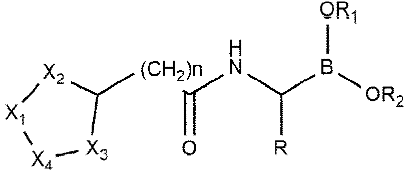 worin X₁ und X₂, die gleich oder verschieden sind, CH, CH₂ sind, die Bindung zwischen X₁ und X₂ gesättigt oder ungesättigt sein kann, worin X₃ und X₄ gleich sind und S sind, n eine ganze Zahl ist, die zwischen 1 und 10 umfasst, R H, Alkyl ist, ausgewählt aus der Gruppe, bestehend aus: Methyl, Ethyl, n-Propyl, n-Butyl, n-Pentyl, n-Hexyl, sec-Butyl, optional substituiert mit einer Gruppe, ausgewählt aus der Gruppe, bestehend aus: Adamantyl, i-Propyl, sec-Butyl, i-Butyl, tert-Butyl, i-Pentyl, Hydroxymethyl, 1-Hydroxyethyl, Sulfanylmethyl, 2-(Methylsulfanyl)ethyl, Carboxymethyl, Carboxyethyl, 4-Aminobutyl, 2-Carbamoylethyl, 2-Carbamoylmethyl, 2-Methyl-3H-imidazol, 3-Methyl-1H-Indol, Alkenyl, ausgewählt aus der Gruppe, bestehend aus: Ethenyl, 1-Propenyl, 2-Propenyl, 1-Butenyl, 2-Butenyl, 3-Butenyl, Isopentenyl, Geranyl, Farnesyl, optional substituiert mit einer Gruppe, ausgewählt aus der Gruppe, bestehend aus: Ethenyl, 2-Propenyl, Isopentenyl, Geranyl, Farnesyl, Aryl, optional substituiert mit einer Gruppe, ausgewählt aus der Gruppe, bestehend aus: Benzyl, 4-Hydroxybenzyl, 3-Alkyl-1H-indol, 2-Methyl-1H-imidazol, 3,4-Dihydroxyphenylmethyl, 4-(2-Aminoethyl)benzen-1,2-diol, 4-Hydroxy-3-methylphenylmethyl, 4-Hydroxy-3-isopropylphenylmethyl, 3,4-Dihydroxyphenylethyl, (3,4-Dihydroxyphenyl)-2-hydroxyethyl, (5-Hydroxy-1H-indol-3-yl)ethyl, zyklischen Verbindungen, die einen oder mehrere Ringe haben, bestehend aus einer Anzahl von Kohlenstoffatomen p, die zwischen 1 und 10 umfasst, optional substituiert, R₁ und R₂, die gleich oder verschieden sind, H, CH₃, Alkyl, Alkenyl, Aryl sind, oder R₁ sind R₂ in einer zyklischen Verbindung, die einen oder mehrere Ringe hat, bestehend aus einer Anzahl von Kohlenstoffatomen m, die zwischen 1 und 10 umfasst, optional substituiert, ihre Stereoisomere, Enantiomere, Konformere und racemische Mischung von verschiedenen Stereoisomeren, zur Verwendung bei der Behandlung von kardiovaskulären Krankheiten, die mit oxidativem Stress verbunden sind.

16. Pharmazeutische Zusammensetzung, umfassend eine pharmazeutisch aktive Menge von Substanzen der Formel (I): 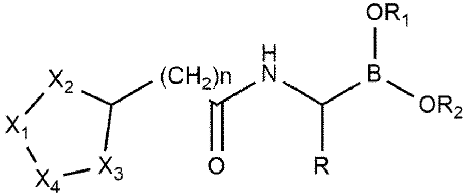 worin X₁ und X₂, die gleich oder verschieden sind, CH, CH₂ sind, die Bindung zwischen X₁ und X₂ gesättigt oder ungesättigt sein kann, worin X₃ und X₄ gleich sind und S sind, n eine ganze Zahl ist, die zwischen 1 und 10 umfasst, R H, Alkyl ist, ausgewählt aus der Gruppe, bestehend aus: Methyl, Ethyl, n-Propyl, n-Butyl, n-Pentyl, n-Hexyl, sec-Butyl, optional substituiert mit einer Gruppe, ausgewählt aus der Gruppe, bestehend aus: Adamantyl, i-Propyl, sec-Butyl, i-Butyl, tert-Butyl, i-Pentyl, Hydroxymethyl, 1-Hydroxyethyl, Sulfanylmethyl, 2-(Methylsulfanyl)ethyl, Carboxymethyl, Carboxyethyl, 4-Aminobutyl, 2-Carbamoylethyl, 2-Carbamoylmethyl, 2-Methyl-3H-imidazol, 3-Methyl-1H-Indol, Alkenyl, ausgewählt aus der Gruppe, bestehend aus: Ethenyl, 1-Propenyl, 2-Propenyl, 1-Butenyl, 2-Butenyl, 3-Butenyl, Isopentenyl, Geranyl, Farnesyl, optional substituiert mit einer Gruppe, ausgewählt aus der Gruppe, bestehend aus: Ethenyl, 2-Propenyl, Isopentenyl, Geranyl, Farnesyl, Aryl, optional substituiert mit einer Gruppe, ausgewählt aus der Gruppe, bestehend aus: Benzyl, 4-Hydroxybenzyl, 3-Alkyl-1H-indol, 2-Methyl-1H-imidazol, 3,4-Dihydroxyphenylmethyl, 4-(2-Aminoethyl)benzen-1,2-diol, 4-Hydroxy-3-methylphenylmethyl, 4-Hydroxy-3-isopropylphenylmethyl, 3,4-Dihydroxyphenylethyl, (3,4-Dihydroxyphenyl)-2-hydroxyethyl, (5-Hydroxy-1H-indol-3-yl)ethyl, zyklischen Verbindungen, die einen oder mehrere Ringe haben, bestehend aus einer Anzahl von Kohlenstoffatomen p, die zwischen 1 und 10 umfasst, optional substituiert, R₁ und R₂, die gleich oder verschieden sind, H, CH₃, Alkyl, Alkenyl, Aryl sind, oder R₁ sind R₂ in einer zyklischen Verbindung, die einen oder mehrere Ringe hat, bestehend aus einer Anzahl von Kohlenstoffatomen m, die zwischen 1 und 10 umfasst, optional substituiert, ihre Stereoisomere, Enantiomere, Konformere und racemische Mischung von verschiedenen Stereoisomeren und geeignete pharmazeutisch akzeptable Hilfsstoffe.

## Revendications

1. Composés de formule (I) : 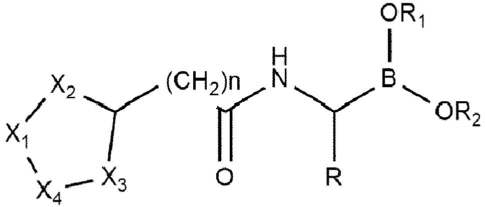 dans lesquels
X₁ et X₂, identiques ou différents, sont CH, CH₂
la liaison entre X₁ et X₂ peut être saturée ou insaturée
X₃ et X₄ sont identiques et sont S
n est un nombre entier compris entre 1 et 10
R est H, un alkyle sélectionné dans le groupe consistant en : un méthyle, un éthyle, un n-propyle, un n-butyle, un n-pentyle, un n-hexyle, un sec-butyle facultativement substitué avec un groupe sélectionné dans le groupe consistant en : un adamantyle, un i-propyle, un sec-butyle, un i-butyle, un tert-butyle, un i-pentyle, un hydroxyméthyle, un 1-hydroxyéthyle, un sulfanylméthyle, un 2-(méthylsulfanyl)éthyle, un carboxyméthyle, un carboxyéthyle, un 4-aminobutyle, un 2-carbamoyléthyle, un 2-carbamoylméthyle, un 2-méthyl-3H-imidazole, un 3-méthyl-1H-indole, un alcényle sélectionné dans le groupe consistant en : un éthényle, un 1-propényle, un 2-propényle, un 1-butényle, un 2-butényle, un 3-butényle, un isopentényle, un géranyle, un farnésyle facultativement substitué avec un groupe sélectionné dans le groupe consistant en : un éthényle, un 2-propényle, un isopentényle, un géranyle, un farnésyle, un aryle facultativement substitué avec un groupe sélectionné dans le groupe consistant en : un benzyle, un 4-hydroxybenzyle, un 3-alkyl-1H-indole, un 2-méthyl-1H-imidazole, un 3,4-dihydroxyphényl-méthyle, un 4-(2-aminoéthyl)benzène-1,2-diol, un 4-hydroxy-3-méthyl-phénylméthyle, un 4-hydroxy-3-isopropyl-phényl-méthyle, un 3,4-dihydroxy-phényl-éthyle, un (3,4-dihydroxy-phényl)-2-hydroxyéthyle, un (5-hydroxy-1H-indol-3-yl)éthyle, des composés cycliques ayant un ou plusieurs cycles, consistant en un nombre d'atomes de carbone p compris entre 1 et 10, facultativement substitués,
R₁ et R₂, identiques ou différents, sont H, CH₃, un alkyle, un alcényle, un aryle ou R₁ sont R₂ dans un composé cyclique ayant un ou plusieurs cycles, consistant en un nombre d'atomes de carbone m compris entre 1 et 10, facultativement substitués, leurs stéréoisomères, énantiomères, conformères et un mélange racémique de différents stéréoisomères.

2. Composés de formule (I) selon la revendication 1 dans lesquels X₁-X₂ est CH₂-CH₂, CH=CH.

3. Composés de formule (I) selon la revendication 1 dans lesquels n est 4.

4. Composés de formule (I) selon la revendication 1 dans lesquels R est H.

5. Composés de formule (I) selon la revendication 1 sélectionnés dans le groupe consistant en les :
5-((S)-1,2-dithiolan-3-yl)-*N-*(((3aR)-3a,5,5-triméthylhexahydro-4,6-méthanebenzo[d][1,3,2,]dioxaborol-2-yl)méthyl) pentanamide
acide (S)-((5-(1,2-dithiolan-3-yl)pentanamide)méthyl)boronique
acide ((S)-1-(5-((R)-1,2-dithiolan-3-yl)pentanamide)éthyl) boronique
acide ((S)-1-(5-((R)-1,2-dithiolan-3-yl)pentanamide)-3-méthylbutyl) boronique
acide ((S)-1-(5-((R)-1,2-dithiolan-3-yl)pentanamide)-2-mercaptoéthyl) boronique
acide ((1S)-1-(5-(1,2-dithiolan-3 -yl)pentanamide)-2-(3,4-dihydroxyphényl)éthyl) boronique
acide ((S)-1-(5-((R)-1,2-dithiolan-3-yl)pentanamide)-2-(4-hydroxy-3-méthylphényl)éthyl) boronique
acide ((S)-1-(5-((R)-1,2-dithiolan-3-yl)pentanamide)-2-(4-hydroxy-3-isopropylphényl)éthyl) boronique
acide ((S)-1-(5-((R)-1,2-dithiolan-3-yl)pentanamide)-3-(3,4-dihydroxyphényl)propyl) boronique
acide ((3S)-1-(5-((R)-1,2-dithiolan-3-yl)pentanamide)-3-(3,4-dihydroxyphényl)-3-hydroxypropyl) boronique
acide ((S)-1-(5-((R)-1,2-dithiolan-3-yl)pentanamide)-2-hydroxyéthyl) boronique
acide (1-(5-((R)-1,2-dithiolan-3-yl)pentanamide)-4-méthylpent-3-én-1-yl) boronique
acide ((S)-1-(5-((R)-1,2-dithiolan-3-yl)pentanamide)-3-(5-hydroxy-1H-indol-3-yl)propyl) boronique
5-((R)-1,2-dithiolan-3-yl)-N-((1S)-1-(3a,5,5-triméthylhexahydro-1H-4,6-méthanebenzo[c]borol-2(3H)-yl)éthyl)pentanamide
5-((R)-1,2-dithiolan-3-yl)-N-((1S)-3-méthyl-1-(3a,5,5-triméthylhexahydro-4,6-méthanebenzo[d][1,3,2]dioxaborol-2-yl)butyl)pentanamide
N-((1S)-2-(3,4-dihydroxyphényl)-1-(3a,5,5-triméthylhexahydro-4,6-méthanebenzo[d][1,3,2]dioxaborol-2-yl)éthyl)-5-((R)-1,2-dithiolan-3-yl)pentanamide
5-((R)-1,2-dithiolan-3-yl)-N-((1S)-2-mercapto-1-(3a,5,5-triméthylhexahydro-4,6-méthanebenzo[d][1,3,2]dioxaborol-2-yl)éthyl)pentanamide
5-((R)-1,2-dithiolan-3-yl)-N-((1S)-2-(4-hydroxy-3-isopropylphényl)-1-(3a,5,5-triméthylhexahydro-4,6-méthanebenzo[d][1,3,2]dioxaborol-2-yl)éthyl)pentanamide
5-((R)-1,2-dithiolan-3-yl)-N-((1S)-2-(4-hydroxy-3-méthylphényl)-1-(3a,5,5-triméthylhexahydro-4,6-méthanebenzo[d][1,3,2]dioxaborol-2-yl)éthyl)pentanamide
N-((3S)-3-(3,4-dihydroxyphényl)-3-hydroxy-1-(3a,5,5-triméthylhexahydro-4,6-méthanebenzo[d][1,3,2]dioxaborol-2-yl)propyl)-5-((R)-1,2-dithiolan-3-yl)pentanamide
N-((1S)-3-(3,4-dihydroxyphényl)-1-(3a,5,5-triméthylhexahydro-4,6-méthanebenzo[d][1,3,2]dioxaborol-2-yl)propyl)-5-((R)-1,2-ditiolan-3-yl)pentanamide
5-((R)-1,2-dithiolan-3-yl)-N-((1S)-2-hydroxy-1-(3a,5,5-triméthylhexahydro-4,6-méthanebenzo[d][1,3,2]dioxaborol-2-yl)éthyl)pentanamide
5-((R)-1,2-dithiolan-3-yl)-N-((1S)-3-(5-hydroxy-1H-indol-3-yl)-1-(3 a, 5,5 - triméthylhexahydro-4,6-methanebenzo[d][1,3,2]dioxaborol-2-yl)propyl)pentanamide
5-((R)-1,2-dithiolan-3-yl)-N-(4-méthyl-1-(3a,5,5-triméthylhexahydro-4,6-méthanebenzo[d][1,3,2]dioxaborol-2-yl)pent-3-én-1-yl)pentanamide.

6. Composés de formule (I) : 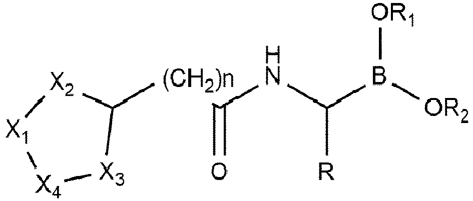 dans lesquels X₁ et X₂, identiques ou différents, sont CH, CH₂, la liaison entre X₁ et X₂ peut être saturée ou insaturée, dans lesquels X₃ et X₄ sont identiques et sont S, n est un nombre entier compris entre 1 et 10, R est H, un alkyle sélectionné dans le groupe consistant en : un méthyle, un éthyle, un n-propyle, un n-butyle, un n-pentyle, un n-hexyle, un sec-butyle facultativement substitué avec un groupe sélectionné dans le groupe consistant en : un adamantyle, un i-propyle, un sec-butyle, un i-butyle, un tert-butyle, un i-pentyle, un hydroxyméthyle, un 1-hydroxyéthyle, un sulfanylméthyle, un 2-(méthylsulfanyl)éthyle, un carboxyméthyle, un carboxyéthyle, un 4-aminobutyle, un 2-carbamoyléthyle, un 2-carbamoylméthyle, un 2-méthyl-3H-imidazole, un 3-méthyl-1H-indole, un alcényle sélectionné dans le groupe consistant en : un éthényle, un 1-propényle, un 2-propényle, un 1-butényle, un 2-butényle, un 3-butényle, un isopentényle, un géranyle, un farnésyle facultativement substitué avec un groupe sélectionné dans le groupe consistant en : un éthényle, un 2-propényle, un isopentényle, un géranyle, un farnésyle, un aryle facultativement substitué avec un groupe sélectionné dans le groupe consistant en : un benzyle, un 4-hydroxybenzyle, un 3-alkyl-1H-indole, un 2-méthyl-1H-imidazole, un 3,4-dihydroxyphényl-méthyle, un 4-(2-aminoéthyl)benzène-1,2-diol, un 4-hydroxy-3-méthyl-phénylméthyle, un 4-hydroxy-3-isopropyl-phényl-méthyle, un 3,4-dihydroxy-phényl-éthyle, un (3,4-dihydroxy-phényl)-2-hydroxyéthyle, un (5-hydroxy-1H-indol-3-yl)éthyle, des composés cycliques ayant un ou plusieurs cycles, consistant en un nombre d'atomes de carbone p compris entre 1 et 10, facultativement substitués, R₁ et R₂, identiques ou différents, sont H, CH₃, un alkyle, un alcényle, un aryle ou R1 sont R₂ dans un composé cyclique ayant un ou plusieurs cycles, consistant en un nombre d'atomes de carbone m compris entre 1 et 10, facultativement substitués, leurs stéréoisomères, énantiomères, conformères et un mélange racémique de différents stéréoisomères, pour leur utilisation en tant que médicament.

7. Composés de formule (I) : 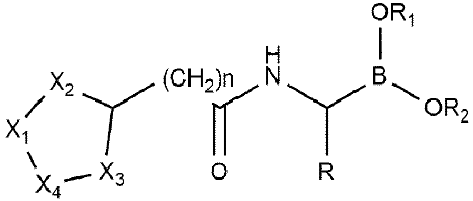 dans lesquels X₁ et X₂, identiques ou différents, sont CH, CH₂, la liaison entre X₁ et X₂ peut être saturée ou insaturée, dans lesquels X₃ et X₄ sont identiques et sont S, n est un nombre entier compris entre 1 et 10, R est H, un alkyle sélectionné dans le groupe consistant en : un méthyle, un éthyle, un n-propyle, un n-butyle, un n-pentyle, un n-hexyle, un sec-butyle facultativement substitué avec un groupe sélectionné dans le groupe consistant en : un adamantyle, un i-propyle, un sec-butyle, un i-butyle, un tert-butyle, un i-pentyle, un hydroxyméthyle, un 1-hydroxyéthyle, un sulfanylméthyle, un 2-(méthylsulfanyl)éthyle, un carboxyméthyle, un carboxyéthyle, un 4-aminobutyle, un 2-carbamoyléthyle, un 2-carbamoylméthyle, un 2-méthyl-3H-imidazole, un 3-méthyl-1H-indole, un alcényle sélectionné dans le groupe consistant en : un éthényle, un 1-propényle, un 2-propényle, un 1-butényle, un 2-butényle, un 3-butényle, un isopentényle, un géranyle, un farnésyle facultativement substitué avec un groupe sélectionné dans le groupe consistant en : un éthényle, un 2-propényle, un isopentényle, un géranyle, un farnésyle, un aryle facultativement substitué avec un groupe sélectionné dans le groupe consistant en : un benzyle, un 4-hydroxybenzyle, un 3-alkyl-1H-indole, un 2-méthyl-1H-imidazole, un 3,4-dihydroxyphényl-méthyle, un 4-(2-aminoéthyl)benzène-1,2-diol, un 4-hydroxy-3-méthyl-phénylméthyle, un 4-hydroxy-3-isopropyl-phényl-méthyle, un 3,4-dihydroxy-phényl-éthyle, un (3,4-dihydroxy-phényl)-2-hydroxyéthyle, un (5-hydroxy-1H-indol-3-yl)éthyle, des composés cycliques ayant un ou plusieurs cycles, consistant en un nombre d'atomes de carbone p compris entre 1 et 10, facultativement substitués, R₁ et R₂, identiques ou différents, sont H, CH₃, un alkyle, un alcényle, un aryle ou R1 sont R₂ dans un composé cyclique ayant un ou plusieurs cycles, consistant en un nombre d'atomes de carbone m compris entre 1 et 10, facultativement substitués, leurs stéréoisomères, énantiomères, conformères et un mélange racémique de différents stéréoisomères, pour leur utilisation en tant que neuroprotecteur.

8. Composés de formule (I) : 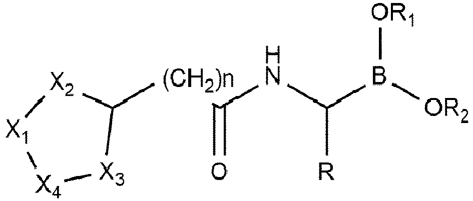 dans lesquels X₁ et X₂, identiques ou différents, sont CH, CH₂, la liaison entre X₁ et X₂ peut être saturée ou insaturée, dans lesquels X₃ et X₄ sont identiques et sont S, n est un nombre entier compris entre 1 et 10, R est H, un alkyle sélectionné dans le groupe consistant en : un méthyle, un éthyle, un n-propyle, un n-butyle, un n-pentyle, un n-hexyle, un sec-butyle facultativement substitué avec un groupe sélectionné dans le groupe consistant en : un adamantyle, un i-propyle, un sec-butyle, un i-butyle, un tert-butyle, un i-pentyle, un hydroxyméthyle, un 1-hydroxyéthyle, un sulfanylméthyle, un 2-(méthylsulfanyl)éthyle, un carboxyméthyle, un carboxyéthyle, un 4-aminobutyle, un 2-carbamoyléthyle, un 2-carbamoylméthyle, un 2-méthyl-3H-imidazole, un 3-méthyl-1H-indole, un alcényle sélectionné dans le groupe consistant en : un éthényle, un 1-propényle, un 2-propényle, un 1-butényle, un 2-butényle, un 3-butényle, un isopentényle, un géranyle, un farnésyle facultativement substitué avec un groupe sélectionné dans le groupe consistant en : un éthényle, un 2-propényle, un isopentényle, un géranyle, un farnésyle, un aryle facultativement substitué avec un groupe sélectionné dans le groupe consistant en : un benzyle, un 4-hydroxybenzyle, un 3-alkyl-1H-indole, un 2-méthyl-1H-imidazole, un 3,4-dihydroxyphényl-méthyle, un 4-(2-aminoéthyl)benzène-1,2-diol, un 4-hydroxy-3-méthyl-phénylméthyle, un 4-hydroxy-3-isopropyl-phényl-méthyle, un 3,4-dihydroxy-phényl-éthyle, un (3,4-dihydroxy-phényl)-2-hydroxyéthyle, un (5-hydroxy-1H-indol-3-yl)éthyle, des composés cycliques ayant un ou plusieurs cycles, consistant en un nombre d'atomes de carbone p compris entre 1 et 10, facultativement substitués, R₁ et R₂, identiques ou différents, sont H, CH₃, un alkyle, un alcényle, un aryle ou R1 sont R₂ dans un composé cyclique ayant un ou plusieurs cycles, consistant en un nombre d'atomes de carbone m compris entre 1 et 10, facultativement substitués, leurs stéréoisomères, énantiomères, conformères et un mélange racémique de différents stéréoisomères, pour leur utilisation en tant qu'agent antiplaquettaire.

9. Composés de formule (I) : 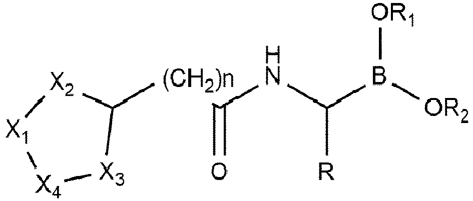 dans lesquels X₁ et X₂, identiques ou différents, sont CH, CH₂, la liaison entre X₁ et X₂ peut être saturée ou insaturée, dans lesquels X₃ et X₄ sont identiques et sont S, n est un nombre entier compris entre 1 et 10, R est H, un alkyle sélectionné dans le groupe consistant en : un méthyle, un éthyle, un n-propyle, un n-butyle, un n-pentyle, un n-hexyle, un sec-butyle facultativement substitué avec un groupe sélectionné dans le groupe consistant en : un adamantyle, un i-propyle, un sec-butyle, un i-butyle, un tert-butyle, un i-pentyle, un hydroxyméthyle, un 1-hydroxyéthyle, un sulfanylméthyle, un 2-(méthylsulfanyl)éthyle, un carboxyméthyle, un carboxyéthyle, un 4-aminobutyle, un 2-carbamoyléthyle, un 2-carbamoylméthyle, un 2-méthyl-3H-imidazole, un 3-méthyl-1H-indole, un alcényle sélectionné dans le groupe consistant en : un éthényle, un 1-propényle, un 2-propényle, un 1-butényle, un 2-butényle, un 3-butényle, un isopentényle, un géranyle, un farnésyle facultativement substitué avec un groupe sélectionné dans le groupe consistant en : un éthényle, un 2-propényle, un isopentényle, un géranyle, un farnésyle, un aryle facultativement substitué avec un groupe sélectionné dans le groupe consistant en : un benzyle, un 4-hydroxybenzyle, un 3-alkyl-1H-indole, un 2-méthyl-1H-imidazole, un 3,4-dihydroxyphényl-méthyle, un 4-(2-aminoéthyl)benzène-1,2-diol, un 4-hydroxy-3-méthyl-phénylméthyle, un 4-hydroxy-3-isopropyl-phényl-méthyle, un 3,4-dihydroxy-phényl-éthyle, un (3,4-dihydroxy-phényl)-2-hydroxyéthyle, un (5-hydroxy-1H-indol-3-yl)éthyle, des composés cycliques ayant un ou plusieurs cycles, consistant en un nombre d'atomes de carbone p compris entre 1 et 10, facultativement substitués, R₁ et R₂, identiques ou différents, sont H, CH₃, un alkyle, un alcényle, un aryle ou R1 sont R₂ dans un composé cyclique ayant un ou plusieurs cycles, consistant en un nombre d'atomes de carbone m compris entre 1 et 10, facultativement substitués, leurs stéréoisomères, énantiomères, conformères et un mélange racémique de différents stéréoisomères, pour leur utilisation en tant qu'agent de chimioprévention.

10. Composés de formule (I) : 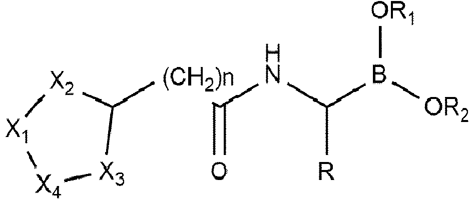 dans lesquels X₁ et X₂, identiques ou différents, sont CH, CH₂, la liaison entre X₁ et X₂ peut être saturée ou insaturée, dans lesquels X₃ et X₄ sont identiques et sont S, n est un nombre entier compris entre 1 et 10, R est H, un alkyle sélectionné dans le groupe consistant en : un méthyle, un éthyle, un n-propyle, un n-butyle, un n-pentyle, un n-hexyle, un sec-butyle facultativement substitué avec un groupe sélectionné dans le groupe consistant en : un adamantyle, un i-propyle, un sec-butyle, un i-butyle, un tert-butyle, un i-pentyle, un hydroxyméthyle, un 1-hydroxyéthyle, un sulfanylméthyle, un 2-(méthylsulfanyl)éthyle, un carboxyméthyle, un carboxyéthyle, un 4-aminobutyle, un 2-carbamoyléthyle, un 2-carbamoylméthyle, un 2-méthyl-3H-imidazole, un 3-méthyl-1H-indole, un alcényle sélectionné dans le groupe consistant en : un éthényle, un 1-propényle, un 2-propényle, un 1-butényle, un 2-butényle, un 3-butényle, un isopentényle, un géranyle, un farnésyle facultativement substitué avec un groupe sélectionné dans le groupe consistant en : un éthényle, un 2-propényle, un isopentényle, un géranyle, un farnésyle, un aryle facultativement substitué avec un groupe sélectionné dans le groupe consistant en : un benzyle, un 4-hydroxybenzyle, un 3-alkyl-1H-indole, un 2-méthyl-1H-imidazole, un 3,4-dihydroxyphényl-méthyle, un 4-(2-aminoéthyl)benzène-1,2-diol, un 4-hydroxy-3-méthyl-phénylméthyle, un 4-hydroxy-3-isopropyl-phényl-méthyle, un 3,4-dihydroxy-phényl-éthyle, un (3,4-dihydroxy-phényl)-2-hydroxyéthyle, un (5-hydroxy-1H-indol-3-yl)éthyle, des composés cycliques ayant un ou plusieurs cycles, consistant en un nombre d'atomes de carbone p compris entre 1 et 10, facultativement substitués, R₁ et R₂, identiques ou différents, sont H, CH₃, un alkyle, un alcényle, un aryle ou R1 sont R₂ dans un composé cyclique ayant un ou plusieurs cycles, consistant en un nombre d'atomes de carbone m compris entre 1 et 10, facultativement substitués, leurs stéréoisomères, énantiomères, conformères et un mélange racémique de différents stéréoisomères, pour leur utilisation en tant qu'agent antitumoral.

11. Composés de formule (I) : 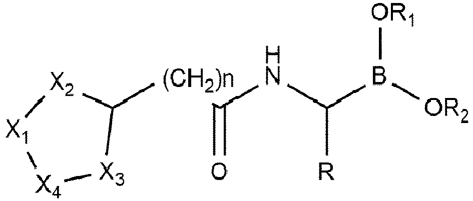 dans lesquels X₁ et X₂, identiques ou différents, sont CH, CH₂, la liaison entre X₁ et X₂ peut être saturée ou insaturée, dans lesquels X₃ et X₄ sont identiques et sont S, n est un nombre entier compris entre 1 et 10, R est H, un alkyle sélectionné dans le groupe consistant en : un méthyle, un éthyle, un n-propyle, un n-butyle, un n-pentyle, un n-hexyle, un sec-butyle facultativement substitué avec un groupe sélectionné dans le groupe consistant en : un adamantyle, un i-propyle, un sec-butyle, un i-butyle, un tert-butyle, un i-pentyle, un hydroxyméthyle, un 1-hydroxyéthyle, un sulfanylméthyle, un 2-(méthylsulfanyl)éthyle, un carboxyméthyle, un carboxyéthyle, un 4-aminobutyle, un 2-carbamoyléthyle, un 2-carbamoylméthyle, un 2-méthyl-3H-imidazole, un 3-méthyl-1H-indole, un alcényle sélectionné dans le groupe consistant en : un éthényle, un 1-propényle, un 2-propényle, un 1-butényle, un 2-butényle, un 3-butényle, un isopentényle, un géranyle, un farnésyle facultativement substitué avec un groupe sélectionné dans le groupe consistant en : un éthényle, un 2-propényle, un isopentényle, un géranyle, un farnésyle, un aryle facultativement substitué avec un groupe sélectionné dans le groupe consistant en : un benzyle, un 4-hydroxybenzyle, un 3-alkyl-1H-indole, un 2-méthyl-1H-imidazole, un 3,4-dihydroxyphényl-méthyle, un 4-(2-aminoéthyl)benzène-1,2-diol, un 4-hydroxy-3-méthyl-phénylméthyle, un 4-hydroxy-3-isopropyl-phényl-méthyle, un 3,4-dihydroxy-phényl-éthyle, un (3,4-dihydroxy-phényl)-2-hydroxyéthyle, un (5-hydroxy-1H-indol-3-yl)éthyle, des composés cycliques ayant un ou plusieurs cycles, consistant en un nombre d'atomes de carbone p compris entre 1 et 10, facultativement substitués, R₁ et R₂, identiques ou différents, sont H, CH₃, un alkyle, un alcényle, un aryle ou R1 sont R₂ dans un composé cyclique ayant un ou plusieurs cycles, consistant en un nombre d'atomes de carbone m compris entre 1 et 10, facultativement substitués, leurs stéréoisomères, énantiomères, conformères et un mélange racémique de différents stéréoisomères, pour leur utilisation en tant qu'antimicrobien.

12. Composés de formule (I) : 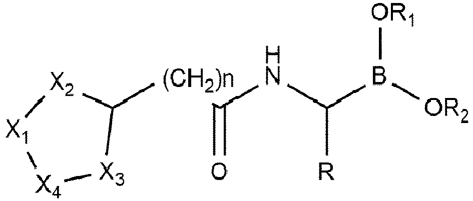 dans lesquels X₁ et X₂, identiques ou différents, sont CH, CH₂, la liaison entre X₁ et X₂ peut être saturée ou insaturée, dans lesquels X₃ et X₄ sont identiques et sont S, n est un nombre entier compris entre 1 et 10, R est H, un alkyle sélectionné dans le groupe consistant en : un méthyle, un éthyle, un n-propyle, un n-butyle, un n-pentyle, un n-hexyle, un sec-butyle facultativement substitué avec un groupe sélectionné dans le groupe consistant en : un adamantyle, un i-propyle, un sec-butyle, un i-butyle, un tert-butyle, un i-pentyle, un hydroxyméthyle, un 1-hydroxyéthyle, un sulfanylméthyle, un 2-(méthylsulfanyl)éthyle, un carboxyméthyle, un carboxyéthyle, un 4-aminobutyle, un 2-carbamoyléthyle, un 2-carbamoylméthyle, un 2-méthyl-3H-imidazole, un 3-méthyl-1H-indole, un alcényle sélectionné dans le groupe consistant en : un éthényle, un 1-propényle, un 2-propényle, un 1-butényle, un 2-butényle, un 3-butényle, un isopentényle, un géranyle, un farnésyle facultativement substitué avec un groupe sélectionné dans le groupe consistant en : un éthényle, un 2-propényle, un isopentényle, un géranyle, un farnésyle, un aryle facultativement substitué avec un groupe sélectionné dans le groupe consistant en : un benzyle, un 4-hydroxybenzyle, un 3-alkyl-1H-indole, un 2-méthyl-1H-imidazole, un 3,4-dihydroxyphényl-méthyle, un 4-(2-aminoéthyl)benzène-1,2-diol, un 4-hydroxy-3-méthyl-phénylméthyle, un 4-hydroxy-3-isopropyl-phényl-méthyle, un 3,4-dihydroxy-phényl-éthyle, un (3,4-dihydroxy-phényl)-2-hydroxyéthyle, un (5-hydroxy-1H-indol-3-yl)éthyle, des composés cycliques ayant un ou plusieurs cycles, consistant en un nombre d'atomes de carbone p compris entre 1 et 10, facultativement substitués, R₁ et R₂, identiques ou différents, sont H, CH₃, un alkyle, un alcényle, un aryle ou R1 sont R₂ dans un composé cyclique ayant un ou plusieurs cycles, consistant en un nombre d'atomes de carbone m compris entre 1 et 10, facultativement substitués, leurs stéréoisomères, énantiomères, conformères et un mélange racémique de différents stéréoisomères, pour leur utilisation en tant qu'antifongique.

13. Composés de formule (I) : 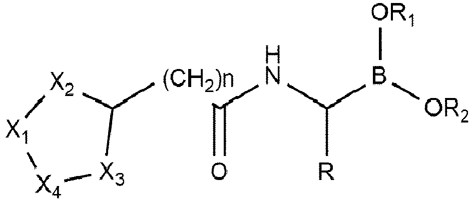 dans lesquels X₁ et X₂, identiques ou différents, sont CH, CH₂, la liaison entre X₁ et X₂ peut être saturée ou insaturée, dans lesquels X₃ et X₄ sont identiques et sont S, n est un nombre entier compris entre 1 et 10, R est H, un alkyle sélectionné dans le groupe consistant en : un méthyle, un éthyle, un n-propyle, un n-butyle, un n-pentyle, un n-hexyle, un sec-butyle facultativement substitué avec un groupe sélectionné dans le groupe consistant en : un adamantyle, un i-propyle, un sec-butyle, un i-butyle, un tert-butyle, un i-pentyle, un hydroxyméthyle, un 1-hydroxyéthyle, un sulfanylméthyle, un 2-(méthylsulfanyl)éthyle, un carboxyméthyle, un carboxyéthyle, un 4-aminobutyle, un 2-carbamoyléthyle, un 2-carbamoylméthyle, un 2-méthyl-3H-imidazole, un 3-méthyl-1H-indole, un alcényle sélectionné dans le groupe consistant en : un éthényle, un 1-propényle, un 2-propényle, un 1-butényle, un 2-butényle, un 3-butényle, un isopentényle, un géranyle, un farnésyle facultativement substitué avec un groupe sélectionné dans le groupe consistant en : un éthényle, un 2-propényle, un isopentényle, un géranyle, un farnésyle, un aryle facultativement substitué avec un groupe sélectionné dans le groupe consistant en : un benzyle, un 4-hydroxybenzyle, un 3-alkyl-1H-indole, un 2-méthyl-1H-imidazole, un 3,4-dihydroxyphényl-méthyle, un 4-(2-aminoéthyl)benzène-1,2-diol, un 4-hydroxy-3-méthyl-phénylméthyle, un 4-hydroxy-3-isopropyl-phényl-méthyle, un 3,4-dihydroxy-phényl-éthyle, un (3,4-dihydroxy-phényl)-2-hydroxyéthyle, un (5-hydroxy-1H-indol-3-yl)éthyle, des composés cycliques ayant un ou plusieurs cycles, consistant en un nombre d'atomes de carbone p compris entre 1 et 10, facultativement substitués, R₁ et R₂, identiques ou différents, sont H, CH₃, un alkyle, un alcényle, un aryle ou R1 sont R₂ dans un composé cyclique ayant un ou plusieurs cycles, consistant en un nombre d'atomes de carbone m compris entre 1 et 10, facultativement substitués, leurs stéréoisomères, énantiomères, conformères et un mélange racémique de différents stéréoisomères, pour leur utilisation dans le traitement du stress oxydatif.

14. Composés de formule (I) : 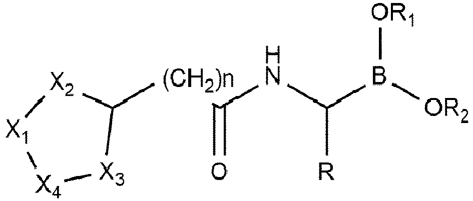 dans lesquels X₁ et X₂, identiques ou différents, sont CH, CH₂, la liaison entre X₁ et X₂ peut être saturée ou insaturée, dans lesquels X₃ et X₄ sont identiques et sont S, n est un nombre entier compris entre 1 et 10, R est H, un alkyle sélectionné dans le groupe consistant en : un méthyle, un éthyle, un n-propyle, un n-butyle, un n-pentyle, un n-hexyle, un sec-butyle facultativement substitué avec un groupe sélectionné dans le groupe consistant en : un adamantyle, un i-propyle, un sec-butyle, un i-butyle, un tert-butyle, un i-pentyle, un hydroxyméthyle, un 1-hydroxyéthyle, un sulfanylméthyle, un 2-(méthylsulfanyl)éthyle, un carboxyméthyle, un carboxyéthyle, un 4-aminobutyle, un 2-carbamoyléthyle, un 2-carbamoylméthyle, un 2-méthyl-3H-imidazole, un 3-méthyl-1H-indole, un alcényle sélectionné dans le groupe consistant en : un éthényle, un 1-propényle, un 2-propényle, un 1-butényle, un 2-butényle, un 3-butényle, un isopentényle, un géranyle, un farnésyle facultativement substitué avec un groupe sélectionné dans le groupe consistant en : un éthényle, un 2-propényle, un isopentényle, un géranyle, un farnésyle, un aryle facultativement substitué avec un groupe sélectionné dans le groupe consistant en : un benzyle, un 4-hydroxybenzyle, un 3-alkyl-1H-indole, un 2-méthyl-1H-imidazole, un 3,4-dihydroxyphényl-méthyle, un 4-(2-aminoéthyl)benzène-1,2-diol, un 4-hydroxy-3-méthyl-phénylméthyle, un 4-hydroxy-3-isopropyl-phényl-méthyle, un 3,4-dihydroxy-phényl-éthyle, un (3,4-dihydroxy-phényl)-2-hydroxyéthyle, un (5-hydroxy-1H-indol-3-yl)éthyle, des composés cycliques ayant un ou plusieurs cycles, consistant en un nombre d'atomes de carbone p compris entre 1 et 10, facultativement substitués, R₁ et R₂, identiques ou différents, sont H, CH₃, un alkyle, un alcényle, un aryle ou R1 sont R₂ dans un composé cyclique ayant un ou plusieurs cycles, consistant en un nombre d'atomes de carbone m compris entre 1 et 10, facultativement substitués, leurs stéréoisomères, énantiomères, conformères et un mélange racémique de différents stéréoisomères, pour leur utilisation dans le traitement de maladies neurodégénératives associées au stress oxydatif.

15. Composés de formule (I) : 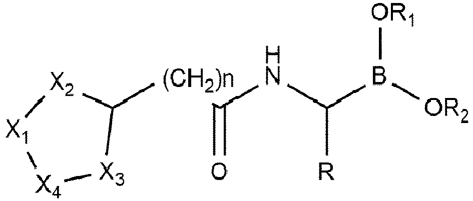 dans lesquels X₁ et X₂, identiques ou différents, sont CH, CH₂, la liaison entre X₁ et X₂ peut être saturée ou insaturée, dans lesquels X₃ et X₄ sont identiques et sont S, n est un nombre entier compris entre 1 et 10, R est H, un alkyle sélectionné dans le groupe consistant en : un méthyle, un éthyle, un n-propyle, un n-butyle, un n-pentyle, un n-hexyle, un sec-butyle facultativement substitué avec un groupe sélectionné dans le groupe consistant en : un adamantyle, un i-propyle, un sec-butyle, un i-butyle, un tert-butyle, un i-pentyle, un hydroxyméthyle, un 1-hydroxyéthyle, un sulfanylméthyle, un 2-(méthylsulfanyl)éthyle, un carboxyméthyle, un carboxyéthyle, un 4-aminobutyle, un 2-carbamoyléthyle, un 2-carbamoylméthyle, un 2-méthyl-3H-imidazole, un 3-méthyl-1H-indole, un alcényle sélectionné dans le groupe consistant en : un éthényle, un 1-propényle, un 2-propényle, un 1-butényle, un 2-butényle, un 3-butényle, un isopentényle, un géranyle, un farnésyle facultativement substitué avec un groupe sélectionné dans le groupe consistant en : un éthényle, un 2-propényle, un isopentényle, un géranyle, un farnésyle, un aryle facultativement substitué avec un groupe sélectionné dans le groupe consistant en : un benzyle, un 4-hydroxybenzyle, un 3-alkyl-1H-indole, un 2-méthyl-1H-imidazole, un 3,4-dihydroxyphényl-méthyle, un 4-(2-aminoéthyl)benzène-1,2-diol, un 4-hydroxy-3-méthyl-phénylméthyle, un 4-hydroxy-3-isopropyl-phényl-méthyle, un 3,4-dihydroxy-phényl-éthyle, un (3,4-dihydroxy-phényl)-2-hydroxyéthyle, un (5-hydroxy-1H-indol-3-yl)éthyle, des composés cycliques ayant un ou plusieurs cycles, consistant en un nombre d'atomes de carbone p compris entre 1 et 10, facultativement substitués, R₁ et R₂, identiques ou différents, sont H, CH₃, un alkyle, un alcényle, un aryle ou R1 sont R₂ dans un composé cyclique ayant un ou plusieurs cycles, consistant en un nombre d'atomes de carbone m compris entre 1 et 10, facultativement substitués, leurs stéréoisomères, énantiomères, conformères et un mélange racémique de différents stéréoisomères, pour leur utilisation dans le traitement de maladies cardiovasculaires associées au stress oxydatif.

16. Composition pharmaceutique comprenant une quantité pharmaceutique active de composés de formule (I) 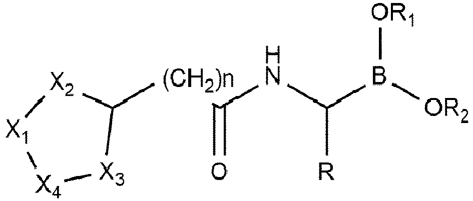 dans laquelle X₁ et X₂, identiques ou différents, sont CH, CH₂, la liaison entre X₁ et X₂ peut être saturée ou insaturée, dans laquelle X₃ et X₄ sont identiques et sont S, n est un nombre entier compris entre 1 et 10, R est H, un alkyle sélectionné dans le groupe consistant en : un méthyle, un éthyle, un n-propyle, un n-butyle, un n-pentyle, un n-hexyle, un sec-butyle facultativement substitué avec un groupe sélectionné dans le groupe consistant en : un adamantyle, un i-propyle, un sec-butyle, un i-butyle, un tert-butyle, un i-pentyle, un hydroxyméthyle, un 1-hydroxyéthyle, un sulfanylméthyle, un 2-(méthylsulfanyl)éthyle, un carboxyméthyle, un carboxyéthyle, un 4-aminobutyle, un 2-carbamoyléthyle, un 2-carbamoylméthyle, un 2-méthyl-3H-imidazole, un 3-méthyl-1H-indole, un alcényle sélectionné dans le groupe consistant en : un éthényle, un 1-propényle, un 2-propényle, un 1-butényle, un 2-butényle, un 3-butényle, un isopentényle, un géranyle, un farnésyle facultativement substitué avec un groupe sélectionné dans le groupe consistant en : un éthényle, un 2-propényle, un isopentényle, un géranyle, un farnésyle, un aryle facultativement substitué avec un groupe sélectionné dans le groupe consistant en : un benzyle, un 4-hydroxybenzyle, un 3-alkyl-1H-indole, un 2-méthyl-1H-imidazole, un 3,4-dihydroxyphényl-méthyle, un 4-(2-aminoéthyl)benzène-1,2-diol, un 4-hydroxy-3-méthyl-phénylméthyle, un 4-hydroxy-3-isopropyl-phényl-méthyle, un 3,4-dihydroxy-phényl-éthyle, un (3,4-dihydroxy-phényl)-2-hydroxyéthyle, un (5-hydroxy-1H-indol-3-yl)éthyle, des composés cycliques ayant un ou plusieurs cycles, consistant en un nombre d'atomes de carbone p compris entre 1 et 10, facultativement substitués, R₁ et R₂, identiques ou différents, sont H, CH₃, un alkyle, un alcényle, un aryle ou R1 sont R₂ dans un composé cyclique ayant un ou plusieurs cycles, consistant en un nombre d'atomes de carbone m compris entre 1 et 10, facultativement substitués, leurs stéréoisomères, énantiomères, conformères et un mélange racémique de différents stéréoisomères et des excipients pharmaceutiquement acceptables adaptés.
